(19)

Europäisches
Patentamt
European
Patent Office
Office européen
des brevets

(11) **EP 2 808 357 A1**

(12) **EUROPEAN PATENT APPLICATION**

(43) Date of publication:
**03.12.2014 Bulletin 2014/49**

(51) Int Cl.:
***C08J 3/12*** *(2006.01)*

(21) Application number: **14170164.9**

(22) Date of filing: **28.05.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **31.05.2013 JP 2013115019**
**28.01.2014 JP 2014012946**

(71) Applicant: **Ricoh Company, Ltd.**
**Tokyo 143-8555 (JP)**

(72) Inventors:
• **Watanabe, Masaki**
**Ohta-ku, Tokyo 143-8555 (JP)**
• **Tanaka, Chiaki**
**Ohta-ku, Tokyo 143-8555 (JP)**
• **Nemoto, Taichi**
**Ohta-ku, Tokyo 143-8555 (JP)**

(74) Representative: **Lamb, Martin John Carstairs**
**Marks & Clerk LLP**
**90 Long Acre**
**London WC2E 9RA (GB)**

(54) **Core-shell type particles and method for producing the same**

(57)    A method for producing core-shell type particles, including:
ring-opening polymerizing a ring-opening polymerizable monomer in a first mixture containing the ring-opening polymerizable monomer, porous particles, and a compressive fluid; and
injecting a second mixture containing a polymer obtained in the ring-opening polymerizing, the porous particles, and the compressive fluid to thereby granulate into the core-shell type particles.

# FIG. 4

EP 2 808 357 A1

**Description**

BACKGROUND OF THE INVENTION

Field of the Invention

The present invention relates to core-shell type particles and a method for producing the core-shell type particles.

Description of the Related Art

Recently, attention has been paid to a particle which is hybrid of an organic material and an inorganic material as a particle having a novel function. Among them, an inorganic particle coated with a polymer may exhibit a novel physical property depending on the type of the polymer.

For example, there has been proposed a surface-modified particle in which a surface of inorganic powder for cosmetics is coated with a silicone polymer (see, e.g., Japanese Patent Application Laid-Open (JP-A) No. 2006-104342).

In contrast, as a particle in which a surface of an organic particle is coated with an inorganic material, there has been proposed a particle which has an improved powder property (e.g., flowability) by sprinkling an external additive (e.g., silica or titanium) on a surface of a toner base particle (see, e.g., Japanese Patent (JP-B) No. 3270198).

Thus, the hybrid of an organic material and an inorganic material can exhibit a novel function which is not exhibited when the organic material or the inorganic material is used alone. Therefore, the hybrid has been developed in a wide range of fields.

A core-shell type particle in which an organic material is used as a coating material and a core particle is composed of an organic material can be utilized in a drug delivery system. In this case, use of a biodegradable polymer as the coating material results in a system in which the coating material on a surface of the core particle decomposes over time to thereby slowly release a drug contained in the core particle for a predetermined time. However, in the case where a core-shell type particle capable of being utilized in the drug delivery system is produced, an organic solvent is generally often used (see, e.g., JP-A No. 11-506744).

The organic solvent used in producing the core-shell type particle is typically removed via a desolvation step, but, disadvantageously, may remain in the order of ppm. When the residual organic solvent is accumulated, it may adversely affect a human body.

Therefore, there has been desired to construct a system without necessity of an organic solvent.

In the core-shell type particle having a core-shell structure in which a drug is coated with a polymer (e.g., a drug delivery system), a core particle is generally often coated by covering a fine structure on the core particle with a coating material. However, in this case, distances from surfaces of the core-shell structure to the core particles vary 1

depending on the position. Therefore, when the coating material decomposes at a constant rate, a time period for which the core particle is exposed is ununiform, potentially leading to unsatisfactory controlled release at a target site. Meanwhile, when the core particle is thinly and uniformly coated with the polymer so as to conform to fine structures on the surface of the core particle, it is believe that the controlled release rate tends to be uniform.

In particular, in the case where the core particle is a porous particle, it dissolves very rapidly when it is brought into contact with human tissue fluid and has increased controlled release efficiency. In such a porous particle, if a surface of fine structure including internal structure (pore) can be thinly coated with a polymer, it is expected that the controlled release rate is uniformized and controlled release efficiency is improved.

SUMMARY OF THE INVENTION

The present invention aims to solve the above existing problems and achieve the following object. That is, an object of the present invention is to provide a method for producing core-shell type particles which is performed without using an organic solvent and which allows core particles to be coated with a polymer so as to conform to fine structures on surfaces of the core particles.

A means for solving the aforementioned problems is as follows:

A method for producing core-shell type particles, including:

ring-opening polymerizing a ring-opening polymerizable monomer in a first mixture containing the ring-opening polymerizable monomer, porous particles, and a compressive fluid; and
injecting a second mixture containing a polymer obtained in the ring-opening polymerizing, the porous particles, and the compressive fluid to thereby granulate into the core-shell type particles.

The present invention can solve the above existing problems, and can provide a method for producing core-shell type particles which is performed without using an organic solvent and which allows core particles to be coated with a polymer so as to conform to fine structures on surfaces of the core particles.

BRIEF DESCRIPTION OF THE DRAWINGS

FIG. 1 is a general phase diagram depicting the state of a substance depending on pressure and temperature conditions.

FIG. 2 is a phase diagram which defines a compressive fluid used in the present embodiment.

FIG. 3 is a schematic diagram illustrating a particle production device used in one embodiment of the present invention.

FIG. 4 is a schematic diagram illustrating a particle production device used in the first method in another embodiment of the present invention (the second embodiment).

FIG. 5 is a schematic diagram illustrating a particle production device used in the first method in another embodiment of the present invention (the second embodiment).

FIG. 6 is a schematic diagram illustrating a particle production device used in the second method in another embodiment of the present invention (the second embodiment).

DETAILED DESCRIPTION OF THE INVENTION

(Production method of core-shell type particles and core-shell type particles)

A method for producing core-shell type particles of the present invention includes a polymerization step and a granulation step; and, if necessary, further includes other steps.

Core-shell type particles of the present invention each contain a core particle which is a porous particle, and a shell layer which coats the core particle.

The core-shell type particles of the present invention can be obtained by, for example, the method for producing core-shell type particles of the present invention.

<Polymerization step>

The polymerization step is not particularly limited and may be appropriately selected depending on the intended purpose, as long as it is a step of ring-opening polymerizing a ring-opening polymerizable monomer in a first mixture containing the ring-opening polymerizable monomer, porous particles, and a compressive fluid.

The polymerization step may be in a batch manner, or a continuous manner.

«First mixture»

The first mixture contains a ring-opening polymerizable monomer, porous particles, and a compressive fluid; preferably contains a catalyst; and, if necessary, further contains other components.

-Ring-opening polymerizable monomer-

The ring-opening polymerizable monomer is not particularly limited and may be appropriately selected depending on the intended purpose, but is preferably a ring-opening polymerizable monomer having a ring structure containing a carbonyl group. The carbonyl group is formed with oxygen, which has high electronegativity, and carbon bonded together with a $\pi$-bond. Because of electrons of the $\pi$-bond, oxygen is negatively polarized, and carbon is positively polarized, and therefore the carbonyl group has enhanced reactivity. In the case where the compressive fluid is carbon dioxide, it is assumed that affinity between the carbon dioxide and the resultant polymer is high, as the carbonyl bond has a structure similar to that of the carbon dioxide. As a result of these functions, the resultant polymer is highly plasticized by the compressive fluid. The ring-opening polymerizable monomer having a ring structure containing a carbonyl group is more preferably a ring-opening polymerizable monomer containing an ester bond.

Examples of the ring-opening polymerizable monomer include cyclic ester and cyclic carbonate.

--Cyclic ester--

The cyclic ester is not particularly limited and may be appropriately selected depending on the intended purpose, but it is preferably a cyclic dimer obtained through dehydration-condensation of an L-form and/or D-form of a compound represented by the following General Formula (1).

R-C*-H(-OH)(-COOH)          General Formula (1)

In the General Formula (1), R denotes a C1-C10 alkyl group, and "C*" denotes an asymmetric carbon.

Examples of the compound represented by the General Formula (1) include enantiomers of lactic acid, enantiomers of 2-hydroxybutanoic acid, enantiomers of 2-hydroxypentanoic acid, enantiomers of 2-hydroxyhexanoic acid, enantiomers of 2-hydroxyheptanoic acid, enantiomers of 2-hydroxy octanoic acid, enantiomers of 2-hydroxynonanoic acid, enantiomers of 2-hydroxydecanoic acid, enantiomers of 2-hydroxyundecanoic acid, and enantiomers of 2-hydroxydodecanoic acid. Among them, enantiomers of lactic acid are preferable since they are highly reactive and readily available.

Examples of other cyclic esters include aliphatic lactone, such as β-propiolactone, β-butyrolactone, γ-butyrolactone, γ-hexanolactone, γ-octanolactone, δ-valerolactone, δ-hexanolactone, δ-octanolactone, ε-caprolactone, δ-dodecanolactone, α-methyl-γ-butyrolactone, β-methyl-δ-valerolactone, glycolide and lactide. Among them, δ-caprolactone is particularly preferable since it is highly reactive and readily available.


--Cyclic carbonate--

The cyclic carbonate is not particularly limited and may be appropriately selected depending on the intended purpose. Examples thereof include ethylene carbonate, and propylene carbonate.

These ring-opening polymerizable monomers may be used alone or in combination.


-Porous particles-

The porous particles are not particularly limited and may be appropriately selected depending on the intended purpose, but are preferably protein, calcium phosphate, metal oxide, or any combination thereof.

The protein is not particularly limited and may be appropriately selected depending on the intended purpose, but is preferably BMP (bone morphogenetic protein), EP4A (EP4 antagonist), FGF-18 (Fibroblast growth factor 18), VEGF (vascular endothelial growth factor), bFGF (basic fibroblast growth factor), or any combination thereof.

The calcium phosphate is not particularly limited and may be appropriately selected depending on the intended purpose, but is preferably hydroxyapatite, α-tricalcium phosphate, β-tricalcium phosphate, tetracalcium phosphate, octacalcium phosphate, or any combination thereof.

The metal oxide is not particularly limited and may be appropriately selected depending on the intended purpose, but is preferably silica, titania, zirconia, zincite, or any combination thereof.

A volume average particle diameter (Dv) of the porous particles is not particularly limited and may be appropriately selected depending on the intended purpose, but is preferably 0.1 μm to 20.0μm, more preferably 0.5 μm to 15.0 μm, particularly preferably 0.5 μm to 10.0 μm.

The volume average particle diameter (Dv) can be measured by means of, for example, MICROTRAC UPA-150 (manufactured by NIKKISO CO., LTD.).

A BET specific surface area of the porous particles is not particularly limited and may be appropriately selected depending on the intended purpose, but is preferably 1 m$^2$/g to 100 m$^2$/g, more preferably 5 m$^2$/g to 50 m$^2$/g, particularly preferably 10 m$^2$/g to 30 m$^2$/g.

The BET specific surface area can be measured by means of, for example, an automatic specific surface area/pore distribution measuring device TRISTAR 3000 (manufactured by SHIMADZU CORPORATION).

An average pore diameter of the porous particles are not particularly limited and may be appropriately selected depending on the intended purpose, but is preferably 20 nm to 200 nm, more preferably 50 nm to 170 nm, particularly preferably 60 nm to 150 nm.

The pore diameter of the porous particle can be determined by observing the porous particle by means of a field emission scanning electron microscope (FE-SEM), followed by measuring a pore in the porous particle for a pore diameter based on the resultant image.

The average pore diameter of the porous particles can be determined from an average value of pore diameters of pores of 100 particles.

An amount of the porous particles contained in the first mixture is not particularly limited and may be appropriately selected depending on the intended purpose, but is preferably 1% by mass to 80% by mass, more preferably 3% by mass to 50% by mass, particularly preferably 5% by mass to 30% by mass, relative to that of the ring-opening polymerizable monomer. When the amount is less than 1% by mass, the shell layer may have an ununiform thickness. When the amount is more than 80% by mass, the core-shell type particles may significantly adhere to each other.

-Compressive fluid-

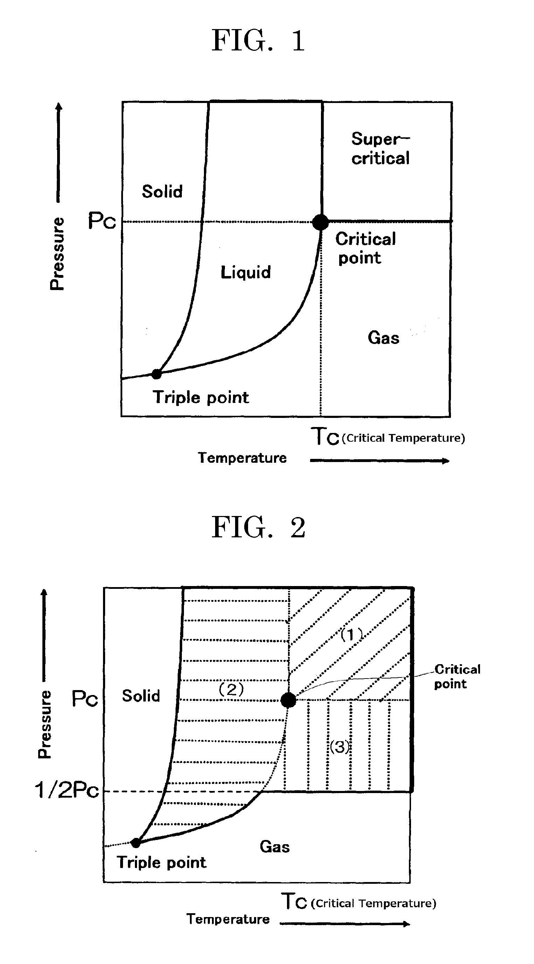

The compressive fluid will be explained with reference to FIGs. 1 and 2. FIG. 1 is a phase diagram depicting the state of a substance depending on pressure and temperature conditions. FIG. 2 is a phase diagram which defines the compressive fluid.

The "compressive fluid" refers to a fluid present in any one of the regions (1), (2) and (3) of FIG. 2 in the phase diagram of FIG. 1.

In such regions, a substance is known to have extremely high density and show different behaviors from those shown at normal temperature and normal pressure. Note that, the substance is a supercritical fluid when it is present in the region (1). The supercritical fluid is a fluid that exists as a non-condensable high-density fluid at temperature and pressure exceeding a limiting point (critical point) at which a gas and a liquid can coexist and that does not condense even when it is compressed. When the substance is in the region (2), the substance is a liquid, but in the present invention, it is a liquefied gas obtained by compressing a substance existing as a gas at normal temperature (25°C) and normal pressure (1 atm). When the substance is in the region (3), the substance is in the state of a gas, but in the present invention, it is a high-pressure gas of which pressure is 1/2 or more of the critical pressure (Pc), i.e. 1/2 Pc or higher.

Examples of a substance constituting the compressive fluid include carbon monoxide, carbon dioxide, dinitrogen oxide, nitrogen, methane, ethane, propane, 2,3-dimethylbutane, and ethylene. Among them, carbon dioxide is preferable because the critical pressure and critical temperature thereof are respectively about 7.4 MPa and about 31°C, and thus carbon dioxide is easily turned into a supercritical state. In addition, carbon dioxide is non-flammable, and therefore it is easily handled. These compressive fluids may be used alone or in combination.

In the case where supercritical carbon dioxide is used as a solvent, it has been conventionally considered that carbon dioxide is not suitable for living anionic polymerization, as it may react with basic and nucleophilic substances (see "The Latest Applied Technology of Supercritical Fluid (CHO RINKAI RYUTAI NO SAISHIN OUYOU GIJUTSU): p. 173, published by NTS Inc. on March 15, 2004). The present inventors have found that, overturning the conventional insight, a polymerization reaction progresses quantitatively in a short time even in the supercritical carbon dioxide by stably coordinating a basic and nucleophilic catalyst with a ring-opening polymerizable monomer to open the ring structure thereof, and, as a result, the polymerization reaction progresses livingly. In the present specification, the term "livingly" means that the reaction progresses quantitatively without a side reaction such as a transfer reaction or termination reaction, so that a molecular weight distribution of the resultant polymer is relatively narrow, and is monodispersible.

--Catalyst--

The catalyst is not particularly limited and may be appropriately selected depending on the intended purpose. Examples thereof include an organic catalyst and a metal catalyst.

---Organic catalyst---

The organic catalyst is not particularly limited and may be appropriately selected depending on the intended purpose. For example, preferable are those containing no metal atom, contributing to a ring-opening polymerization reaction of the ring-opening polymerizable monomer, and capable of being removed and regenerated through a reaction with alcohol after an active intermediate product is formed from the ring-opening polymerizable monomer.

When a ring-opening polymerizable monomer containing an ester bond is polymerized, for example, the organic catalyst is preferably a (nucleophilic) compound having basicity and serving as a nucleophilic agent, more preferably a compound containing a nitrogen atom, and particularly preferably a cyclic compound containing a nitrogen atom. Such a compound is not particularly limited and may be appropriately selected depending on the intended purpose. Examples thereof include cyclic monoamine, cyclic diamine (e.g., a cyclic diamine compound having an amidine skeleton), a cyclic triamine compound having a guanidine skeleton, a heterocyclic aromatic organic compound containing a nitrogen atom, and N-heterocyclic carbene. Note that, a cationic organic catalyst may be used for the ring-opening polymerization, but the cationic organic catalyst withdraws hydrogen atoms from the polymer backbone (back-biting). As a result, the resultant polymer tends to have a wide molecular weight distribution, and it is difficult to obtain a high molecular weight polymer.

Examples of the cyclic monoamine include quinuclidine.

Examples of the cyclic diamine include 1,4-diazabicyclo-[2.2.2]octane (DABCO), and 1,5-diazabicyclo(4,3,0)-5-nonene.

Examples of the cyclic diamine compound having an amidine skeleton include 1,8-diazabicyclo[5.4.0]undec-7-ene (DBU), and diazabicyclononene.

Examples of the cyclic triamine compound having a guanidine skeleton include 1,5,7-triazabicyclo[4.4.0]dec-5-ene (TBD), and diphenyl guanidine (DPG).

Examples of the heterocyclic aromatic organic compound containing a nitrogen atom include N,N-dimethyl-4-aminopyridine (DMAP), 4-pyrrolidinopyridine (PPY), pyrrocoline, imidazole, pyrimidine and purine.

Examples of the N-heterocyclic carbene include 1,3-di-tert-butylimidazol-2-ylidene (ITBU).

Among them, DABCO, DBU, DPG, TBD, DMAP, PPY, and ITBU are preferable, as they have high nucleophilicity without being greatly affected by steric hindrance, or they have such boiling points that they can removed under the reduced pressure.

Among these organic catalysts, for example, DBU is liquid at room temperature, and has a boiling point. In the case where such organic catalyst is selected, the organic catalyst can be removed substantially quantitatively from the resultant polymer by treating the polymer under the reduced pressure. Note that, the type of the organic solvent, or whether or not a removal treatment is performed, is determined depending on an intended use of a polymer product.

---Metal catalyst---

The metal catalyst is not particularly limited and may be appropriately selected depending on the intended purpose. Examples thereof include a tin compound, an aluminum compound, a titanium compound, a zirconium compound, and an antimony compound.

Examples of the tin compound include tin octylate, tin dibutyrate, and tin di(2-ethylhexanoate).

Examples of the aluminum compound include aluminum acetylacetonate, and aluminum acetate.

Examples of the titanium compound include tetraisopropyl titanate, and tetrabutyl titanate.

Example of the zirconium compound includes zirconium isopropoxide.

Example of the antimony compound includes antimony trioxide.

The type and the amount of the catalyst cannot be collectively determined as they vary depending on a combination of the compressive fluid and the ring-opening polymerizable monomer, but the amount thereof is preferably 0.01 mol% to 15 mol%, more preferably 0.1 mol% to 1 mol%, and particularly preferably 0.3 mol% to 0.5 mol%, relative to that of the ring-opening polymerizable monomer. When the amount thereof is less than 0.01 mol%, the catalyst is deactivated before the completion of the polymerization reaction, and, as a result, a polymer having a target molecular weight cannot be obtained in some cases. When the amount thereof is greater than 15 mol%, it may be difficult to control the polymerization reaction.

As a catalyst used in the polymerization step, the organic catalyst (organic catalyst containing no metal atom) is suitably used in an application in which the resultant product is needed to be safe and stable.

-Other components-

Examples of the other components include an initiator and an additive.

--Initiator--

The initiator is used for controlling a molecular weight of a polymer obtained through the ring-opening polymerization.

The initiator is not particularly limited and may be appropriately selected depending on the intended purpose. For example, in the case where the initiator is alcohol-based, it may be aliphatic mono- or poly-hydric alcohol, and it may be saturated or unsaturated.

Examples of the initiator include monoalcohol, dialcohol, polyhydric alcohol and lactate ester. Examples of the monoalcohol include methanol, ethanol, propanol, butanol, pentanol, hexanol, heptanol, nonanol, decanol, lauryl alcohol, myristyl alcohol, cetyl alcohol, and stearyl alcohol. Examples of the polyhydric alcohol include dialcohols such as ethylene glycol, 1,2-propanediol, 1,3-propanediol, 1,3-butanediol, 1,4-butanediol, hexanediol, nonanediol, tetramethylene glycol, and polyethylene glycol; glycerol, sorbitol, xylitol, ribitol, erythritol, and triethanol amine. Examples of the lactate ester include methyl lactate, and ethyl lactate. These may be used alone or in combination.

Also, a polymer containing a terminal alcohol residue such as polycaprolactonediol or polytetramethylene glycol may be used as the initiator. A use of such polymer enables to synthesize diblock copolymers and triblock copolymers.

Additionally, an inorganic material containing a terminal alcohol residue such as hydroxyapatite may also be used as the initiator, which results in an organic-inorganic complex.

An amount of the initiator used in the polymerization step may be appropriately adjusted depending on a target molecular weight, but it is preferably 0.1 mol% to 5 mol% relative to that of the ring-opening polymerizable monomer. In order to prevent the polymerization from being initiated unevenly, the initiator is preferably sufficiently mixed with the monomer before the monomer is brought into contact with the catalyst.

--Additive--

If necessary, an additive may be added in the polymerization step. Examples thereof include a surfactant and an antioxidant.

The surfactant is suitably selected from those dissolved in the compressive fluid and having an affinity with both of the compressive fluid and the ring-opening polymerizable monomer. A use of the surfactant can give effects that the polymerization reaction is allowed to progress uniformly, and the resultant polymer has a narrow molecular weight distribution. In the case where the surfactant is used, the surfactant may be added to the compressive fluid, or to the ring-opening polymerizable monomer. In the case where carbon dioxide is used as the compressive fluid, for example, a surfactant having a group having affinity with carbon dioxide and a group having affinity with the monomer in a molecule thereof may be used. Examples of such surfactant include a fluorosurfactant and a silicone surfactant.

<<Polymer>>

In the polymerization step, the ring-opening polymerizable monomer is allowed to ring-opening polymerize to thereby obtain a polymer.

The weight average molecular weight of the polymer can be adjusted by adjusting an amount of the initiator. The weight average molecular weight thereof is not particularly limited and may be appropriately selected depending on the intended purpose, but it is preferably 5,000 to 200,000, more preferably 12,000 to 200,000, particularly preferably 30,000 to 60,000. When the weight average molecular weight thereof is greater than 200,000, productivity is deteriorated because of the increased viscosity, which may be economically disadvantageous. When the weight average molecular weight thereof is smaller than 5,000, it may not be preferable because the resultant polymer has insufficient strength.

The value (Mw/Mn) obtained by dividing the weight average molecular weight Mw of the polymer with the number average molecular weight Mn thereof is not particularly limited and may be appropriately selected depending on the intended purpose, but it is preferably 1.0 to 2.5, more preferably 1.0 to 2.0. When the value thereof is greater than 2.0, the polymerization reaction is very likely to progress ununiformly, and therefore it is difficult to control physical properties of the polymer.

In the polymerization step, it is possible to carry out the polymerization reaction at a low temperature as the compressive fluid is used. Accordingly, a depolymerization reaction can be significantly prevented compared to a conventional melt polymerization, which results in the polymerization rate of 96 mol% or greater, preferably 98 mol% or greater. When the polymerization rate is less than 96 mol%, the polymer product has unsatisfactory thermal property, and therefore it may be necessary to separately provide an operation for removing the ring-opening polymerizable monomer. Note that, the polymerization rate is a ratio of a ring opening polymerizable monomer contributed to polymer production, relative to a ring-opening polymerizable monomer used as raw materials. The amount of the ring-opening polymerizable monomer contributed to polymer production can be obtained by deducting the amount of an unreacted ring-opening polymerizable monomer (amount of residual ring-opening polymerizable monomer) from the amount of a polymer product.

The polymer is preferably a copolymer having two or more polymer segments. Moreover, the polymer is preferably a stereo complex. Taking stereo complex polylactic acid as an example, the term "stereo complex" means a polylactic acid composition which contains a poly-D-lactic acid component and a poly-L-lactic acid component, and has a stereo complex crystal, where the degree of the crystallinity of stereo complex represented by the following formula (i) is 90% or higher. The degree of the crystallinity of stereo complex (S) is determined from heat of melting of a homocrystal of polylactic acid ($\Delta$Hmh) measured at a temperature lower than 190°C and heat of melting of a stereo complex crystal of polylactic acid ($\Delta$Hmsc) measured at a temperature of 190°C or higher as measured by a differential scanning calorimeter (DSC) using the following formula (i):

$$(S) = [\Delta Hmsc/(\Delta Hmh + \Delta Hmsc)] \times 100 \qquad \cdots (i)$$

The polymer may be or may not be chemically bound to the porous particles in core-shell type particles.

<Granulation step>

The granulation step is not particularly limited and may be appropriately selected depending on the intended purpose, as long as it is a step of injecting a second mixture containing the polymer obtained in the polymerization step, the porous particles, and the compressive fluid to thereby granulate into core-shell type particles.

<<Second mixture>>

The second mixture contains the polymer obtained in the polymerization step, the porous particles, and the compressive fluid; and, if necessary, further contains other components.

The second mixture is a mixture obtained through the polymerization step from the first mixture.

The second mixture may contain a second compressive fluid different from the compressive fluid (the first compressive fluid).

-Second compressive fluid-

The second compressive fluid is mixed with a mixture obtained through the polymerization step from the first mixture, before the mixture is injected.

The second compressive fluid is not particularly limited and may be appropriately selected depending on the intended purpose. For example, the above-described compressive fluid may be used. A compressive fluid containing a substance which has the maximum inversion temperature of 800 K or lower (e.g., oxygen and nitrogen) may also be used. Among them, a nitrogen-containing compressive fluid is preferred.

Here, the "nitrogen-containing" means containing nitrogen molecules, and the air can also be said as "nitrogen-containing." The nitrogen has the maximum inversion temperature of 620 K, which is lower than that of a substance such as carbon dioxide (maximum inversion temperature: 1,500 K). Therefore, reduction in temperature due to the Joule-Thomson effect in the case where the pressure of nitrogen is reduced is smaller than the case where the pressure of the carbon dioxide is reduced. When the maximum inversion temperature of the second compressive fluid is excessively high, such as in the case of the carbon dioxide, cooling due to the Joule-Thomson effect becomes excessive when a mixture (e.g., the second mixture) is injected, so that the mixture is solidified before the mixture is formed into particles. As a result, fibrous or cohesion products may be contaminated. When the cooling is excessive, moreover, the mixture is solidified inside a nozzle, which is used for injecting the mixture, and therefore particles having small particle diameters with a narrow particle size distribution cannot be produced over a long period of time.

The second compressive fluid may also be used together with an entrainer (co-solvent). Example of the entrainer includes an organic solvent. Examples of the organic solvent include alcohols such as methanol, ethanol, and propanol; ketones such as acetone and methyl ethyl ketone; toluene, ethyl acetate, and tetrahydrofurane.

-Injection-

The granulation step is preferably performed by injecting the second mixture via a nozzle.

An inner diameter of the nozzle is not particularly limited and may be appropriately selected depending on the intended purpose, but is preferably 10 $\mu$m to 200 $\mu$m, more preferably 50 $\mu$m to 150 $\mu$m, particularly preferably 75 $\mu$m to 125 $\mu$m. When the inner diameter of the nozzle is smaller than 10 $\mu$m, the nozzle may be clogged. When the inner diameter of the nozzle is more than 200 $\mu$m, the mass flow rate is increased, potentially leading to unstable injection. When the inner diameter of the nozzle falls within the particularly preferable range, it is advantageous in terms of injection stability.

A temperature of the second mixture to be injected is not particularly limited and may be appropriately selected depending on the intended purpose, but is preferably 30°C to 50°C.

A pressure of the second mixture to be injected is not particularly limited and may be appropriately selected depending on the intended purpose, but is preferably 10 MPa to 100MPa, more preferably 25 MPa to 75 MPa, particularly preferably 40 MPa to 60 MPa.

For example, the core-shell type particles can be obtained as follows. The ring-opening polymerizable monomer is allowed to ring-opening polymerize in a mixture containing the ring-opening polymerizable monomer, the porous particles, and the compressive fluid. The resultant mixture optionally mixed with the second compressive fluid. Thereafter, the resultant mixture is injected via a nozzle under an atmospheric pressure to thereby obtain the core-shell type particles. The core-shell type particles contain the polymer. In the core-shell type particles, surfaces of the porous particles are coated with the polymer, and porosity is maintained.

The core-shell type particles each contain a core particle and a shell layer which coats the core particle.

The core particles contain the porous particles.

The shell layer contains the polymer.

A volume average particle diameter (Dv) of the core-shell type particles is not particularly limited and may be appropriately selected depending on the intended purpose, but is preferably 0.1 $\mu$m to 20.0 $\mu$m, more preferably 0.5 $\mu$m to 15.0 $\mu$m, particularly preferably 0.5 $\mu$m to 10.0 $\mu$m.

The volume average particle diameter (Dv) can be measured, for example, by means of MICROTRAC UPA-150 (manufactured by NIKKISO CO., LTD.).

An average thickness of the shell layers in the core-shell type particles is not particularly limited and may be appropriately selected depending on the intended purpose, but is preferably 10 nm to 3 $\mu$m, more preferably 50 nm to 2 $\mu$m, particularly preferably 100 nm to 1 $\mu$m. When the average thickness is less than 10 nm, the shell layer may be scraped off. When the average thickness is more than 3 $\mu$m, it may be difficult to form a shell layer having a uniform thickness. When the average thickness falls within the particularly preferable range, a shell layer having a uniform thickness is easily formed. Therefore, in the case where the resultant core-shell type particles are used in DDS (Drug Delivery System), it is advantageous in that stable controlled-releasability can be ensured.

The thickness of the shell layer in the core-shell type particle can be measured by means of a ruler based on an image obtained through observation of a cross-section of the core-shell type particle by a scanning electron microscope.

The average thickness can be determined from an average value of thicknesses of the shell layer at 100 points.

The BET specific surface area of the core-shell type particle is not particularly limited and may be appropriately selected depending on the intended purpose, but is preferably 1 m$^2$/g to 100 m$^2$/g, more preferably 5 m$^2$/g to 50 m$^2$/g, particularly preferably 10 m$^2$/g to 30 m$^2$/g.

The BET specific surface area can be measured by means of, for example, an automatic specific surface area/pore distribution measuring device TRISTAR 3000 (manufactured by SHIMADZU CORPORATION).

A difference between the BET specific surface area of the core-shell type particle and that of the porous particle is not particularly limited and may be appropriately selected depending on the intended purpose, but is preferably 1.5 m$^2$/g or less, more preferably 1.0 m$^2$/g or less, particularly preferably 0.5 m$^2$/g or less in terms of an absolute value. When the difference is more than 1.5m$^2$/g in terms of an absolute value, step coverage on a pore is impaired, so that a shell layer having a uniform thickness is difficult to be formed. In the case where the resultant core-shell type particles are used in DDS (Drug Delivery System), stable controlled-releasability may not be ensured. Meanwhile, when the difference falls within the particularly preferable range in terms of an absolute value, it is advantageous in that such stable controlled-releasability can be ensured.

An average pore diameter of the core-shell type particles is not particularly limited and may be appropriately selected depending on the intended purpose, but is preferably 10 nm to 200 nm, more preferably 20 nm to 130 nm, particularly preferably 40 nm to 100 nm.

The pore diameter and the average pore diameter can be determined in the same manner as in the pore diameter and the average pore diameter of the porous particles.

A difference between the average pore diameter of the core-shell type particles and that of the porous particles is not particularly limited and may be appropriately selected depending on the intended purpose, but is preferably 100 nm or less, more preferably 80 nm or less, particularly preferably 50 nm or less in terms of an absolute value. When the difference is more than 100 nm in terms of an absolute value, step coverage on a pore is impaired, so that a shell layer having a uniform thickness is difficult to be formed. In the case where the resultant core-shell type particles are used in DDS (Drug Delivery System), stable controlled-releasability may not be ensured. Meanwhile, when the difference falls within the particularly preferable range in terms of an absolute value, it is advantageous in that such stable controlled-releasability can be ensured.

<Particle production device>

Next, one exemplary particle production device used in producing core-shell type particles will be described with reference to figures.

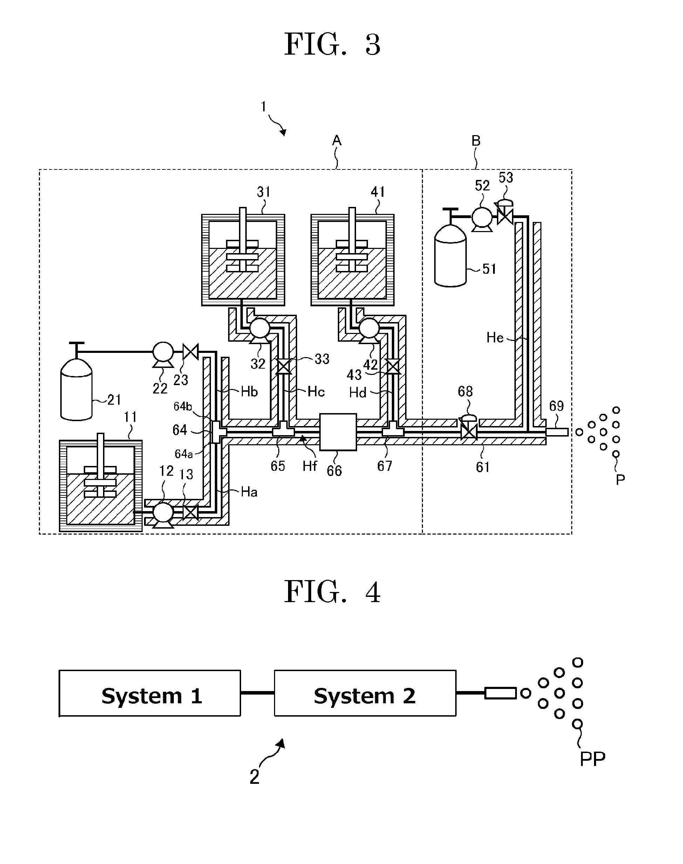

FIG. 3 is a schematic diagram showing one exemplary particle production device used in producing core-shell type particles of the present embodiments. A particle production device of the present embodiment includes an inlet configured to introduce a ring-opening polymerizable monomer and a compressive fluid inlet configured to introduce a compressive fluid, which are provided at one end of a path through which a mixture containing the ring-opening polymerizable monomer and porous particles or a mixture containing the resultant polymer and porous particles is passed. At the other end of the particle production device, provided is a nozzle configured to inject the mixture containing the polymer and the porous particles. A catalyst inlet configured to introduce a catalyst is provided between the one end and the other end.

In a particle production device 1, a tank 11 equipped with a temperature regulator, a pump 12, and a valve 13 are provided, which are connected with each other via a pipe Ha to thereby constitute a first path. Additionally, in the particle production device 1, a bomb 21, a pump 22, and a valve 23 are provided, which are connected with each other via a pipe Hb to thereby constitute a second path. In the particle production device 1, a catalyst tank 31 equipped with a temperature regulator, a pump 32, and a valve 33 are provided, which are connected with each other via a pipe Hc to thereby constitute a third path. In the particle production device 1, an additive tank 41 equipped with a temperature regulator, a pump 42, and a valve 43 are provided, which are connected with each other via a pipe Hd to thereby constitute a fourth path. In the particle production device 1, a bomb 51, a pump 52, and a back pressure valve 53 are provided, which are connected with each other via a pipe He to thereby constitute a fifth path. In the particle production device 1, a reaction container 66, a back pressure valve 68, and a nozzle 69 are provided, which are connected with each other via a pipe Hf to thereby constitute a sixth path. Note that, the pipe Hf is one exemplary path through which the mixture containing the ring-opening polymerizable monomer and the porous particles or the mixture containing the resultant polymer and the porous particles is passed

An end of each of the first path, the second path, and the sixth path in the particle production device 1 are connected with each other by a mixer 64. The third path and the sixth path in the particle production device 1 are connected with each other via a mixer 65, as shown in FIG. 3. The fourth path and the sixth path in the particle production device 1 are connected with each other via a mixer 67, as shown in FIG. 3. The fifth path and the sixth path in the particle production device 1 are connected with each other, as shown in FIG. 3.

In the present embodiment, the term "pipe H" refers to any of pipes (Ha, Hb, Hc, Hd, He, and Hf). The pipe H is not particularly limited, as long as it allows each raw material, the compressive fluid, or the mixture containing the resultant polymer and the porous particles to pass therethrough. However, the pipe H is preferably an ultra high pressure pipe. Note that, the pipe H is regulated in temperature by a heater 61. Each of pumps, valves, mixers, and a reaction container is also regulated in temperature.

The tank 11 provided in the first path is a device configured to store the ring-opening polymerizable monomer and the porous particles, and heat-melt the ring-opening polymerizable monomer. The ring-opening polymerizable monomer to be stored may be in a solid form at room temperature, as long as it is heat-molten and liquefied through control by the temperature regulator installed in the tank 11. The tank 11 may contain a stirrer, which allows the ring-opening polymerizable monomer to be molten more rapidly. An initiator may be added to the tank 11 in advance. Additionally, an additive which does not participate in the reaction may be added to the tank 11 in advance. The pump 12 is a device configured to pump out the ring-opening polymerizable monomer in a molten form and the porous particles in the tank 11 with the application of pressure. The valve 13 is a device configured to open or close the path between the pump 12 and the mixer 64 to thereby adjust the flow rate or shut off the flow therein.

The bomb 21 provided in the second path is a pressure resistant container configured to store and supply a substance which is turned into the first compressive fluid in the mixer 64 (e.g., carbon dioxide). The substance to be stored in the bomb 21 is preferably air, nitrogen, or carbon dioxide, more preferably carbon dioxide, from the viewpoints of cost and safety. Note that, the substance to be stored in the bomb 21 may be in a gas or liquid form, as long as it is turned into the first compressive fluid upon the application of heat or pressure in the path leading to the mixer 64. The pump 22 is a device configured to pump out the substance stored in the bomb 21 with the application of pressure. The valve 23 is a device configured to open or close the path between the pump 22 and the mixer 64 to thereby adjust the flow rate shut off the flow therein.

The mixer 64 contains an inlet 64a configured to introduce the ring-opening polymerizable monomer and the porous particles, and a compressive fluid inlet 64b configured to introduce the compressive fluid. Thus, the mixer 64 brings raw materials supplied from the first path (e.g., the ring-opening polymerizable monomer, the porous particles, and the initiator) into contact with the first compressive fluid supplied from the second path to thereby mix together, followed by sending out to the sixth path. In the present embodiment, the mixer 64 contains a turbulent mixing mechanism in order to homogeneously mix the first compressive fluid, the ring-opening polymerizable monomer, and the porous particles. Specific examples of the mixer 64 include a known T-shape coupling, a swirl mixer which actively utilizes a swirl flow, a static mixer, and a central collision mixer in which two fluids are brought into collision in a mixing part. In the case where the ring-opening polymerizable monomer in the molten form supplied from the first path has significantly high viscosity, a double-screw stirrer equipped with a power source may be used.

In the mixer 64, the ring-opening polymerizable monomer is molten or dissolved by bringing the ring-opening polymerizable monomer into contact with the porous particles and the compressive fluid. In the presence of the ring-opening polymerizable monomer or the polymer product and the compressive fluid, "molten" means the state in which the ring-opening polymerizable monomer or the polymer product is plasticized and liquefied as well as swollen, by being brought in contact with the compressive fluid. Meanwhile, "dissolved" means the state in which the ring-opening polymerizable monomer or the polymer product is dissolved into the compressive fluid. In the case where the ring-opening polymerizable monomer is molten or dissolved, a molten phase or a dissolved phase is formed, respectively. In order to allow the reaction to proceed uniformly, it is preferred that the molten phase does not coexist with a fluid phase, and either the molten phase or the fluid phase is formed. Additionally, in the present embodiment, in order to allow the reaction to proceed in the state in which a ratio of the raw materials to the compressive fluid is high, the reaction preferably proceeds in the presence of only the molten phase.

The catalyst tank 31 provided in the third path stores a catalyst. The catalyst tank 31 is equipped with a temperature regulator, which heat-melts the catalyst in the solid form. Note that, the catalyst may be liquefied by adding an organic solvent thereto or bringing it into contact with the compressive fluid in the catalyst tank 31. In the case where the catalyst is liquid, the temperature regulator is not necessary. The catalyst tank 31 may be equipped with a stirrer, which allows the catalyst to be liquefied more rapidly. The pump 32 is a device configured to apply pressure to the liquefied catalyst in the catalyst tank 31 to thereby send out to the sixth path. The mixer 65 is not particularly limited and may be the same as or different from the mixer 64, as long as it can homogeneously mix the catalyst with a mixture containing the first compressive fluid.

A reaction container 66 is a pressure resistant container configured to allow the ring-opening polymerizable monomer to ring-opening polymerize. The shape of the reaction container 66 may be tank-like or tubular, but is preferably tubular from the viewpoint of a decreased dead space. Note that, the reaction container 66 may be contain a gas outlet configured to remove exhalation. Further, the reaction container 66 contains a heater configured to heat supplied raw materials. Additionally, the reaction container 66 may contain a stirrer configured to stir the raw materials and the first compressive fluid. In the case where the reaction container 66 contains the stirrer, the reaction can proceed more uniformly and quantitatively as the polymer can be prevented from sedimentating by the action of a difference in density between the ring-opening polymerizable monomer and the polymer product. The stirrer in the reaction container 66 is preferably a double- or multi-screw stirrer having screws engaging with each other, stirring elements of 2-flights (rectangle), stirring elements of 3-flights (triangle), or circular or multi-leaf shaped (clover shaped) stirring wings, from the viewpoint of self-cleaning. In the case where the raw materials including the catalyst are sufficiently mixed in advance, a motionless mixer, which divides the flow and compounds (recombines) the flows in multiple stages by a guide device, can also be used as the stirrer. Examples of the motionless mixer include multiflux batch mixers disclosed in Japanese Patent Application Publication (JP-B) Nos. 47-15526, 47-15527, 47-15528, and 47-15533; a Kenics-type mixer disclosed in JP-A No. 47-33166; and mixers without a movable part similar to those listed. In the case where the reaction container 66 is not equipped with a stirrer, a tubular reactor or an ultra high pressure pipe is suitably used as the reaction container 66.

FIG. 3 illustrates an embodiment where one reaction container is used, but a device with two or more reaction containers can be also used. In the case where a plurality of reaction containers are used, reaction (polymerization) conditions per reaction container, i.e., conditions, such as temperature, concentration of the catalyst, pressure, average retention time, and stirring speed, can be the same as in the case where only one reaction container is used, but they are preferably optimized per reaction container corresponding to progress of the polymerization. Note that, it is not very good idea that an excessively large number of containers are connected to give many stages, as it may extend a reaction time, or complicate the device. The number of stages is preferably 1 to 4, particularly preferably 1 to 3. In the case where the polymerization is performed with only one reaction container, the degree of polymerization of the resultant polymer or an amount of residual monomer in the polymer is generally unstable and tends to vary, which is unsuitable for industrial productions. This is probably resulted from instability caused by coexistence of a polymerization material having the melt viscosity of about several poises to about several tens poises with the polymer product having the melt viscosity of about several thousands poises in the same container. In contrast, in the present embodiment, a viscosity difference in the reaction container 66 (also referred to as polymerization system) can be reduced by melting (liquefying) the raw materials and the polymer product, so that the polymer can be stably produced even when the number of the stages can be reduced compared to that in a conventional polymerization reactor.

An additive tank 41 provided in the fourth path may be equipped with a temperature reregulate and is a device configured to heat-melt the additive. The additive tank 41 may be equipped with a stirrer, which allows the additive to be molten more rapidly. A pump 42 is a device configured to apply pressure to the additive in the molten form in the additive tank 41 to thereby send out to the sixth path. The fourth path may not be used in the case where the additive is not necessary.

A mixer 67 is not particularly limited and may be the same as or different from the mixer 64 or 65, as long as it can homogeneously mix the additive with a mixture containing the polymer produced in the reaction container 66.

A bomb 51 is a pressure resistant container configured to store and supply a substance which is turned into the second compressive fluid in the fifth path. The substance to be stored in the bomb 51 is preferably air, nitrogen, argon, helium, or carbon dioxide from the viewpoint of safety, more preferably air, nitrogen, or carbon dioxide in view of cost as well as safety. Note that, the substance to be stored in the bomb 51 may be in the gas or liquid form, as long as it is turned into the second compressive fluid upon the application of heat or pressure in the fifth path.

A pump 52 is a device configured to send out the second compressive fluid stored in the bomb 51 to the sixth path. A back pressure valve 53 is a device configured to open or close the path between the pump 52 and the sixth path to thereby adjust the flow rate of the second compressive fluid or shut off the flow thereof. If necessary, a pressure accumulator may be installed between the pump 52 and the back pressure valve 53. Note that, the compressive fluid heated at the heater 61 is cooled at an exit of the nozzle 69 due to the Joule-Thomson effect. Therefore, the compressive fluid is preferably in the supercritical fluid state, i.e., (1) in the phase diagram of FIG. 2.

A back pressure valve 68 is a device configured to open or close the path between the mixer 67 and the nozzle 69 to thereby adjust the flow rate or pressure of the mixture obtained in the mixer 67, or shut off the flow thereof.

The nozzle 69 in the particle production device 1 is a device configured to supply and, at the same time, inject the second compressive fluid supplied from the fifth path to a mixture containing the first compressive fluid. The mixture injected from the nozzle 69 can be prevented from decreasing in pressure by supplying the second compressive fluid to the mixture, leading to improved processability. Therefore, even when the polymer has high molecular weight, core-shell type particles can be produced.

The type of the nozzle 69 is not particularly limited, but is preferably a direct injection nozzle. The diameter of the nozzle 69 is not particularly limited, as long as pressure upon injection can be maintained at a constant level. However, when the diameter of the nozzle is excessively large, the mixture is increased in viscosity due to excessively low pressure upon injection, which may make it difficult to produce fine particles. Additionally, it may be necessary to increase the size of the pump 52 in order to maintain pressure inside the nozzle 69. Meanwhile, when the diameter of the nozzle is excessively small, the nozzle 69 is likely to be clogged with the second mixture, which may make it difficult to obtain desired core-shell type particles. Therefore, the upper limit of the diameter of the nozzle is not limited, and the lower limit thereof is preferably 5 $\mu$m or larger, more preferably 20 $\mu$m or larger, particularly preferably 50 $\mu$m or larger.

<<Polymerization step>>

Firstly, the pumps 12 and 22 are activated to open the valves 13 and 23, to thereby bring the ring-opening polymerizable monomer and the porous particles into contact with the first compressive fluid in the mixer 64 and mix together. Thus, the ring-opening polymerizable monomer is molten in the presence of the first compressive fluid to thereby obtain a mixture Y1. Next, the pump 32 is activated to open the valve 33, followed by mixing the mixture Y1 with the catalyst in the mixer 65 to thereby obtain a mixture Y2. Note that, in the mixtures Y1 and Y2, the ring-opening polymerizable monomer is in the molten form by the action of the compressive fluid. In the present embodiment, the ring-opening polymerizable monomer is molten in the presence of the first compressive fluid, and then the catalyst is added thereto. In the conventional art, the timing for adding a catalyst has not been discussed in association with a ring-opening polymerization of a ring-opening polymerizable monomer using a compressive fluid. In the present embodiment, in the course of the ring-opening polymerization, the catalyst is added after the mixture Y1 is obtained by sufficiently mixing the first compressive fluid with raw materials such as the ring-opening polymerizable monomer, the porous particles, and the initiator in the mixer 64 because of the high activity of the catalyst. When the catalyst is added in the state where the ring-opening polymerizable monomer in the mixture Y1 is not sufficiently molten, the reaction may progress ununiformly. In the case where the ring-opening polymerizable monomer or the catalyst is solid at normal temperature, the catalyst is molten by heating in the tank 11 or the catalyst tank 31. Besides the heating, a method in which an organic solvent is added to the catalyst or a method in which the catalyst is brought into contact with the compressive fluid may be used. In the case where the mixers 64 and 65 contain the stirrer, the raw materials and the first compressive fluid may be stirred.

The supplying speed of the pumps 12 and 32 is adjusted based on a target mass ratio of the ring-opening polymerizable monomer and the catalyst so that the mass ratio is kept constant. A total mass of the ring-opening polymerizable monomer, the porous particles, and the catalyst supplied per unit time by the pumps 12 and 32 (feeding amount of raw materials (g/min)) is adjusted based on desirable physical properties of a polymer or a reaction time. Similarly, the feeding amount of the first compressive fluid supplied by the pump 22 (g/min) is also adjusted based on desirable physical properties of a polymer or a reaction time.

A ratio of the supplied amount of the raw material to that of the first compressive fluid (feeding amount of the raw material/feeding amount of the first compressive fluid in mass ratio) is preferably 1 or more, more preferably 3 or more, further preferably 5 or more, particularly preferably 10 or more. The upper limit of the feeding ratio is preferably 1,000 or less, more preferably 100 or less, particularly preferably 50 or less.

By setting the feeding ratio to 1 or greater, the reaction progresses with the high concentration (i.e., high solid content) of the raw materials and the polymer product in the reaction container 66. The solid content in this polymerization system is largely different from a solid content in a polymerization system where polymerization is performed by dissolving a small amount of a ring-opening polymerizable monomer in a significantly large amount of a compressive fluid in accordance with a conventional production method. The production method of the present embodiment is characterized by that a polymerization reaction progresses efficiently and stably even in a polymerization system having a high solid content. Note that, in the present embodiment, the feeding ratio may be less than 1. In this case, the resultant polymer and core-shell type particles are not problematic in quality, but economic efficiency is deteriorated. When the feeding ratio is greater than 1,000, there is a possibility that the compressive fluid may insufficiently melt the ring-opening polymerizable monomer therein, and the intended reaction may progress ununiformly.

The mixture Y2 obtained in the mixer 65 is optionally sufficiently mixed by the stirrer of the reaction container 66, and then heated to the predetermined temperature by a heater. Thus, the ring-opening polymerizable monomer is allowed to ring-opening polymerize in the reaction container 66 in the presence of the catalyst.

The temperature during ring-opening polymerization of the ring-opening polymerizable monomer (polymerization reaction temperature) is not particularly limited, but it is 40°C or higher, preferably 50°C or higher, more preferably 60°C or higher. When the polymerization reaction temperature is lower than 40°C, depending on the type of the ring-opening polymerizable monomer, it may take a long time to melt the ring-opening polymerizable monomer in the compressive fluid, or the ring-opening polymerizable monomer may be insufficiently molten, or the catalyst may be deteriorated in activity. As a result, the reaction speed tends to decrease during the polymerization, and therefore the polymerization reaction may not be able to proceed quantitatively.

The upper limit of the polymerization reaction temperature is not particularly limited, but it is whichever is higher of either 170°C or a temperature that is higher than the melting point of the ring-opening polymerizable monomer by 30°C. The upper limit of the polymerization reaction temperature is preferably whichever is higher of either 150°C or the melting point of the ring-opening polymerizable monomer. The upper limit of the polymerization reaction temperature is more preferably whichever is higher of either 130°C or temperature that is lower than the melting point of the ring-opening polymerizable monomer by 20°C. When the polymerization reaction temperature is higher than the temperature that is higher than the melting point of the ring-opening polymerizable monomer by 30°C, a depolymerization reaction, which is a reverse reaction of the ring-opening polymerization, tends to be caused equilibrately, and therefore the polymerization reaction is difficult to proceed quantitatively. In the case of a ring-opening polymerizable monomer having low melting point, such as a ring opening polymerizable monomer that is liquid at room temperature, the polymerization reaction temperature may be a temperature that is higher than the melting point of the ring opening polymerizable monomer by 30°C or more in order to enhance the activity of the catalyst. In this case, the polymerization reaction temperature is not particularly limited, but may be a temperature that is lower than the melting point of the polymer product, preferably 170°C or lower.

Note that, the polymerization reaction temperature is controlled by a heater provided in the reaction container 66 or by heating from the outside of the reaction container 66.

In a conventional method for producing a polymer using supercritical carbon dioxide, a ring-opening polymerizable monomer has been allowed to polymerize using a large amount of supercritical carbon dioxide as supercritical carbon dioxide has low ability of dissolving a polymer. According to the present embodiment, a ring-opening polymerizable monomer can be allowed to ring-opening polymerize with a high concentration, which has not been realized in a conventional art, in the course of production of core-shell type particles using a compressive fluid. In this case, the internal pressure of the reaction container 66 is increased in the presence of the compressive fluid, and thus a glass transition temperature (Tg) of a polymer product is decreased. As a result, the polymer product is decreased in viscosity, and therefore the ring-opening reaction progresses uniformly even in the state where the concentration of the polymer is high. In the present embodiment, in the case where the ring-opening polymerizable monomer is continuously brought into contact with the first compressive fluid to thereby melt therein, the polymer product tends not to vary in concentration in the reaction system.

In the present embodiment, the polymerization reaction time (the average retention time in the reaction container 66) is set depending on a target molecular weight of a polymer product, but typically is preferably 1 hour or shorter, more preferably 45 min or shorter, particularly preferably 30 min or shorter. According to the method in the present embodiment, the polymerization reaction time may be 20 min or shorter. This polymerization reaction time is short, which has not been realized in polymerization of a ring-opening polymerizable monomer in a compressive fluid.

The pressure during the polymerization, i.e., the pressure of the first compressive fluid, may be the pressure at which the first compressive fluid supplied from the bomb 21 is a liquefied gas ((2) in the phase diagram of FIG. 2), or a high pressure gas ((3) in the phase diagram of FIG. 2), but it is preferably the pressure at which the first compressive fluid is a supercritical fluid ((1) in the phase diagram of FIG. 2). By making the first compressive fluid into a supercritical fluid, melting of the ring-opening polymerizable monomer is accelerated, so that the polymerization reaction can progress uniformly and quantitatively. Note that, in the case where carbon dioxide is used as the first compressive fluid, the pressure is 3.7 MPa or higher, preferably 5 MPa or higher, more preferably 7.4 MPa, i.e., the critical pressure or higher, in view of reaction efficiency and polymerization rate. In the case where carbon dioxide is used as the compressive fluid, moreover, the temperature thereof is preferably 25°C or higher from the same reasons as described above.

The moisture content in the reaction container 66 is not particularly limited and may be appropriately selected depending on the intended purpose, but is preferably 4 mol% or less, more preferably 1 mol% or less, particularly preferably 0.5 mol% or less, relative to that of the ring-opening polymerizable monomer. When the moisture content is greater than 4 mol%, it may be difficult to control a molecular weight of a polymer product as the moisture itself acts as the initiator. In order to control the moisture content in the polymerization system, an operation for removing moisture contained in the ring-opening polymerizable monomer and other raw materials may be optionally provided as a pretreatment.

In the reaction container 66, the ring-opening polymerizable monomer in the molten form is reacted to thereby obtain a polymer in the molten form. In this case, the viscosity of a mixture Y3 containing the polymer and the first compressive fluid is not particularly limited, as long as it is the viscosity at which the mixture can be injected by the nozzle 69. However, the viscosity is preferably as low as possible because the mixture can be injected even when the diameter of the nozzle becomes smaller and the mixture can be formed into fine particles. Note that, in the mixture Y3, the polymer is molten in the compressive fluid.

If necessary, an additive may be added to a mixture containing the polymer produced in the reaction container 66. In the case where the additive participates in the reaction, the pump 42 is activated to open the valve 43, to thereby mix the mixture containing the polymer produced in the reaction container 66 with the additive in the mixer 67. In the case where the additive does not participate in the reaction, the additive may be added to the tank 11 at the same time with the ring-opening polymerizable monomer in advance. Here, in the case where the additive is solid at normal temperature, the temperature regulator in the additive tank 41 is activated to melt the additive by heating. Besides the heating, a method in which an organic solvent is added to the additive or a method in which the additive is brought into contact with the compressive fluid may be used. In the mixer 67 contains the stirrer, the mixture containing the polymer produced in the reaction container 66 with the additive may be stirred.

<<Granulation step>>

Subsequently, a granulation step in a method for producing the core-shell type particles of the present embodiment will be described. This granulation step is a step of supplying and, at the same time, injecting the second compressive fluid to the mixture Y3 containing the polymer obtained in the polymerization step to thereby granulate the mixture Y3.

One example using the particle production device 1 of FIG. 3 will be described. A bomb 51 stores nitrogen which is one exemplary substance to be turned into the second compressive fluid in the second path. A pump 52 applies pressure to the nitrogen stored in the bomb 51, and supplies it to the sixth path via a back pressure valve 53. If necessary, a pressure accumulator may be installed between the pump 52 and the back pressure valve 53. The pressure applied by the pump 52 or the pressure accumulator is not particularly limited and may be appropriately selected depending on the intended purpose, but is preferably 1 MPa or greater, more preferably 10 MPa to 200 MPa, and particularly preferably 31 MPa to 100 MPa. When the pressure applied to the compressive fluid is smaller than 1 MPa, it may not be able to plasticize the polymer to the degree sufficient for granulating the polymer when the polymer is fluidized. It is not problematic however high the pressure is, but a more durable device is required corresponding to an increase of pressure, which increase an equipment cost.

The nitrogen supplied from the pump 52 is heated by the heater 61 to thereby be turned into the compressive fluid. The set temperature of the heater 61 is not particularly limited, as long as it is a temperature at which a supplied substance can be turned into the compressive fluid.

Next, the mixture Y3 containing the polymer product, the porous particles, and the first compressive fluid is supplied from the reaction container 66 to the nozzle 69 by opening a back pressure valve 68. Thus, the mixture Y3 supplied from the reaction container 66 is continuously brought into contact with the second compressive fluid supplied from the bomb 51 to thereby continuously inject them from the nozzle 69 under ambient pressure by the action of a difference in pressure. Thus, the mixture Y3 can be injected from the nozzle 69 while the second compressive fluid is supplied. Note that, in the mixture Y3, the polymer is molten in the compressive fluid.

In this case, the solid content of the mixture to be injected is reduced by supplying the second compressive fluid, and therefore the viscosity of the mixture Y3 can be further reduced. As a result, the mixture Y3 to be injected is controlled to a constant temperature. Additionally, the injection speed (outlet linear speed) increases, and shearing force to the mixture Y3 increases due to an increase in the outlet linear speed. Since nitrogen is used as the second compressive fluid, moreover, a decrease in temperature due to the Joule-Thomson effect, which is caused along with the change in the pressure in proximity to the nozzle 69, is inhibited, so that the nozzle 69 is less likely to clog. The mixture Y3 injected from the nozzle 69 is formed into particles P, followed by being solidified. In this case, uniform fine particles without cohesion can be obtained over a long period of time because of a synergistic effect of a decreased viscosity and a decreased solid content of the mixture. Moreover, produced particles can be also uniformly stabilized in shape.

According to the production method of the present embodiment, a polymer melt can be formed at a temperature almost equal to the melting point of the ring-opening polymerizable monomer by the ring-opening polymerizable monomer is brought into contact with the compressive fluid to allow the ring-opening polymerizable monomer to ring-opening polymerize. In contrast, as a conventional method, in the case where a polymer is heated to melt, and then mixed with the compressive fluid to thereby granulate, the polymer must be heated to a temperature equal to or higher than the melting point of the polymer. According to the production method of the present embodiment, it is possible to carry out a granulation at low temperature. Accordingly, a depolymerization reaction of the polymer can be significantly prevented compared to a conventional production method, which results in the polymerization rate of the ring-opening polymerizable monomer of 96 mol% or greater, preferably 98 mol% or greater in core-shell type particles P. When the polymerization rate is less than 96 mol%, the polymer has unsatisfactory thermal property, and therefore it may be necessary to separately provide an operation for removing the ring-opening polymerizable monomer. Note that, in the present embodiment, the polymerization rate is a ratio of a ring opening polymerizable monomer contributed to polymer production, relative to a ring-opening polymerizable monomer used as raw materials. The amount of the ring-opening polymerizable monomer contributed to polymer production can be obtained by deducting the amount of an unreacted ring-opening polymerizable monomer (amount of residual ring-opening polymerizable monomer) from the amount of a polymer product.

In the present invention, in the case where the ring-opening polymerization is performed without using a metal catalyst to thereby produce core-shell type particles, the resultant core-shell type particles are substantially free of the metal catalyst and an organic solvent, and contain only a very small amount of a residual monomer. Therefore, the core-shell type particles are excellent in safety and stability. Accordingly, the core-shell type particles obtained in the present embodiment is widely used in applications such as commodities, medicines, cosmetics, and electrophotographic toners.

Note that, as used herein, "metal catalyst" means a catalyst which is used for a ring-opening polymerization and contains metal, and "substantially free of metal catalyst" means an amount of the metal catalyst contained in a polymer is below the detection limit as detected by a known analysis method such as an inductively coupled plasma spectrometry, an atomic spectrophotometry, or a colorimetry.

Additionally, as used herein, "organic solvent" means a solvent which is organic matter used for a ring-opening polymerization, and into which the polymer obtained in the ring-opening polymerization is dissolved, and "substantially free of organic solvent" means an amount of the organic solvent contained in the polymer is below the detection limit as measured by the following method.

(Measurement method of residual organic solvent)

To 1 part by mass of a polymer serving as a measuring object, 2 parts by mass of 2-propanol was added, followed by dispersing for 30 min by ultrasonic waves. Then, the resultant is stored in a refrigerator (5°C) for 1 day or longer, to thereby extract an organic solvent in the polymer. The thus obtained supernatant fluid was analyzed by a gas chromatography (GC-14A, SHIMADZU), to quantify the organic solvent and the residual monomer in the polymer. Thus, a concentration of the organic solvent is determined. The measuring conditions of this analysis are as follows.

Device: GC-14A SHIMADZU
Column: CBP20-M 50-0.25
Detector: FID
Injection amount: 1 $\mu$L to 5 $\mu$L
Carrier gas: He, 2.5 kg/cm$^2$
Flow rate of hydrogen: 0.6 kg/cm$^2$
Flow rate of air: 0.5 kg/cm$^2$
Chart speed: 5 mm/min
Sensitivity: Range 101 $\times$ Atten 20
Column temperature: 40°C
Injection temperature: 150°C

[Second embodiment] (Applied example)

Subsequently, a second embodiment, which is an applied example of the first embodiment, will be described. In the second embodiment, a polymer complex is synthesized by appropriately adjusting the timing for adding a plurality of ring-opening polymerizable monomers. Note that, in the present embodiment, the term "complex" means a copolymer having two or more polymer segments obtained by polymerizing monomers with a plurality of systems, or a mixture of two or more polymers obtained by polymerizing monomers with a plurality of systems. Two synthesis methods of a stereo complex, which is one example of the complex, will be described.

<First method>

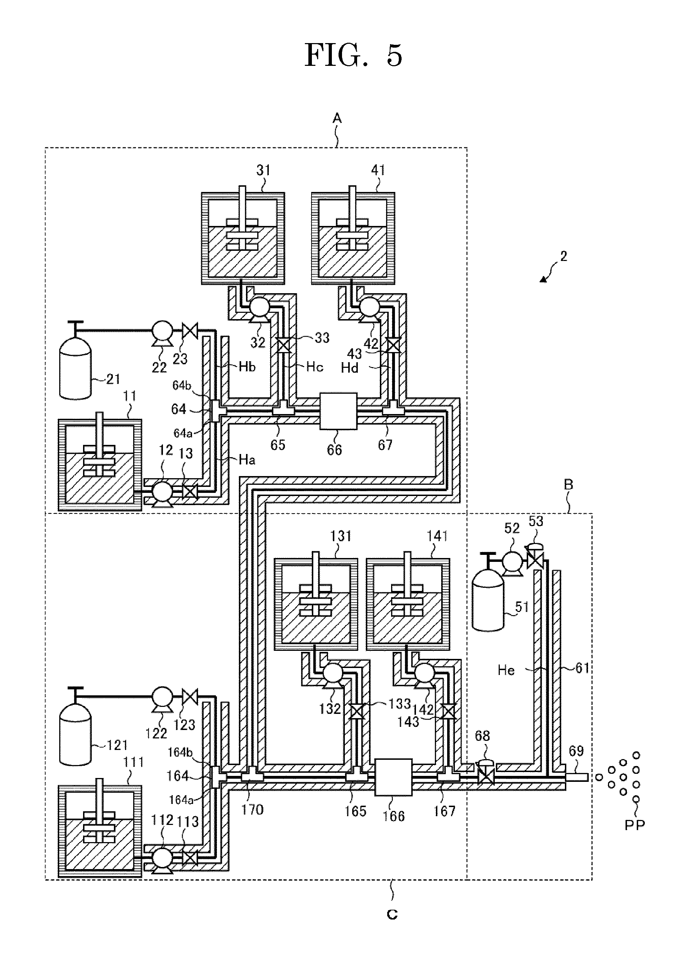

First, a first method will be described with reference to FIGs. 4 and 5. FIGs. 4 and 5 are each a schematic diagram illustrating a particle production system for use in the first method. In the first method, a polymer is produced in System 1 in the particle production device 2 of FIG. 4 in the same manner as in the production method of the first embodiment. The polymer serving as an intermediate is brought into contact with a newly introduced second ring-opening polymerizable monomer in System 2, followed by continuously mixing in the presence of the first compressive fluid, to thereby produce core-shell type particles PP of a complex product (a final polymer). Note that, a complex product having three or more segments may be obtained by tandemly repeating a system similar to System 2 in the particle production device 2 of FIG. 4.

Subsequently, specific example of the particle production device 2 will be described with reference to FIG. 5. The particle production device 2 contains, as System 1, a configuration which is the same as Section A in the particle production device 1 of the first embodiment (see FIG. 3); and, as System 2, Section C and a configuration which is the same as Section B in the particle production device 1 of the first embodiment (see FIG. 3). Note that, Section A and Section B in the particle production device 2 will not described in detail, because they have the same configurations as Section A and Section B in the particle production device 1, respectively.

In the particle production device 2, Section C has the same configuration as Section A, except that the mixer 170 is provided which is placed between the mixer 164 and mixer 165, and which is configured to mix a polymer containing the first compressive fluid produced in Section A.

The tank 111 in Section C of System 2 has the same configuration as the tank 11 in Section A of System 1, except that it stores the second ring-opening polymerizable monomer. The bomb 121, the catalyst tank 131, and the additive tank 141 in Section C of System 2 have the same configurations as the bomb 21, the catalyst tank 31, and the additive tank 41 in Section A of the System 1, respectively. The compressive fluid, catalyst, and additive to be stored may be the same as or different from those in Section A. The pumps (112, 122, 132, and 142), the valves (113, 123, 133, and 143), the mixers (164, 165, and 167), and the reaction container 166 in Section C of System 2 have the same configurations as the pumps (12, 22, 32, and 42), the valves (13, 23, 33, and 43), the mixer (64, 65, and 67), and the reaction container 66 in Section A of System 1, respectively. Note that, the mixer 164 contains an inlet 164a configured to introduce the ring-opening polymerizable monomer and a compressive fluid inlet 164b configured to introduce the compressive fluid.

The mixer 170 is a device configured to mix a mixture Y1-2 supplied from the mixer 164 and containing the second ring-opening polymerizable monomer with a polymer mixture Y3 supplied from Section A of System 1 and serving as an intermediate, to thereby produce a mixture Y4. The mixer 170 is not particularly limited and may be the same as or different from the mixer 164, except that it can homogeneously mix the mixture Y1-2 containing the second ring-opening polymerizable monomer with the polymer mixture Y3 supplied from System 1 and obtained through the ring-opening polymerization of the first ring-opening polymerizable monomer.

In the first method, the first ring-opening polymerizable monomer (e.g., L-lactide) is polymerized in the reaction container 66. After the completion of the reaction quantitatively, an optical isomer (e.g., D-lactide) of the ring-opening polymerizable monomer, which is one example of the second ring-opening polymerizable monomer, is added to the reaction container 166 to thereby further carry out a polymerization reaction. As a result, a stereo block copolymer is obtained. A mixture Y5 containing he resultant stereo block copolymer is turned into core-shell type particles PP which is composed of the complex through the granulation step which is the same as in the first embodiment. This method is effective because racemization hardly occurs, because the reaction progresses at a temperature equal to or lower than the melting point of the ring-opening polymerizable monomer with little amount of residual ring-opening polymerizable monomer, and because the particles PP is produced by an efficient one-step reaction.

Note that, in the first method, the porous particles serving as raw material may be stored in the tank 11, the tank 111, or both thereof.

<Second method>

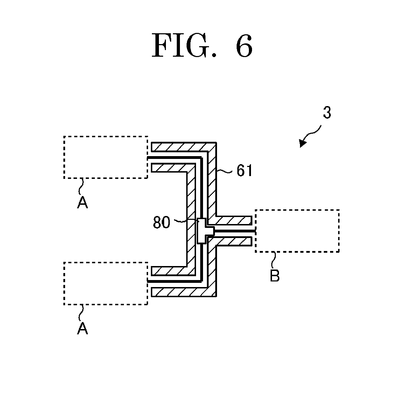

Subsequently, the second method will be described with reference to FIG. 6. FIG. 6 is a schematic diagram illustrating a particle production device 3 for use in the second method. In the second method, a complex product is produced by continuously mixing a plurality of polymers each produced by the production method of the first embodiment, in the presence of the first compressive fluid. The plurality of the polymers are, for example, products each obtained by polymerizing ring-opening polymerizable monomers that are optically isomeric to each other. The particle production device 3 contains a polymerization section in which configurations being the same as Section A in the particle production device 1 of the first embodiment are disposed in parallel, the mixer 80, and a granulation section which has the same configuration as Section B in the particle production device 1 of the first embodiment.

Note that, in the second method, the porous particles serving as raw material may be stored in one or both of tanks 11 in two Sections A.

The mixer 80 is not particularly limited, as long as it can mix a plurality of polymers supplied from Section A of each of Systems. Examples thereof include a known T-shape coupling, a swirl mixer which actively utilizes a swirl flow, a static mixer, and a central collision mixer in which two fluids are brought into collision in a mixing part. It is desired to control a temperature of the mixer 80 by a heater or a jacket. A temperature (mixing temperature) during mixing the polymers in the mixer 80 can be set in the same manner as in setting the polymerization reaction temperature in the reaction container 66 in Section A of each of Systems. Note that, the mixer 80 may contain a mechanism for separately supplying a compressive fluid to the polymers to be mixed.

An inlet of the mixer 80 is connected to an outlet of Section A of each of Systems via an ultra high pressure pipe which is pressure resistant. The outlet of Section A means an outlet of the reaction container 66 or the mixer 67. In any case, a polymer produced in Section A of each of Systems or a mixture containing the polymer and porous particles can be supplied to the mixer 80 with the polymer being in the molten state without turning back to the atmospheric pressure. As a result, it is possible to mix two or more polymers at lower temperature in the mixer 80, as the polymers are decreased in viscosity, in the presence of the compressive fluid. Note that, FIG. 6 illustrates an example where two Sections A are provided parallel by providing the ultra high pressure pipe, but three or more Sections A may be provided parallel by providing a plurality of connectors. In this case, the porous particles serving as raw material may be stored in one or more of tanks 11 in three Sections A.

In the second method, an L-form monomer and D-form monomer (e.g., lactide) are each separately polymerized in the presence of the first compressive fluid in the polymerization step in Section A in advance. The polymers obtained through polymerization are mixed together in the presence of the first compressive fluid to thereby obtain a complex. Generally, a polymer such as polylactic acid often decomposes by re-heating, even when the polymer contains extremely little amount of residual ring-opening polymerizable monomer. The second method is effective because, similarly to the first method, racemization or thermal deterioration can be inhibited by mixing low viscous polylactic acids in the molten form at the temperature equal to or lower than the melting point in the presence of the first compressive fluid.

Note that, in the first method and the second method, methods for producing a stereo complex by separately polymerizing ring-opening polymerizable monomers which are optically isomeric to each other were described. However, ring-opening polymerizable monomers for use in the present embodiment are not necessarily optically isomeric to each other. Moreover, by combining the first method and the second method, block copolymers for forming a stereo complex can be mixed.

<Effect of embodiments>

In accordance with the method for producing core-shell type particles of the present embodiment, it is possible to provide core-shell type particles being excellent from the viewpoints of low cost, low environmental load, energy saving, and resource saving, and having excellent moldability and thermal stability, because of the following reasons.

(1) A reaction progresses at a lower temperature compared to a melt polymerization method in which a reaction progresses at high temperature (e.g., higher than 150°C).

(2) As the reaction progresses at a lower temperature, a side reaction hardly occurs, and thus a polymer can be obtained at high yield relative to an amount of a ring-opening polymerizable monomer added (namely, an amount of unreacted ring-opening polymerizable monomer is small). Accordingly, a purification step for removing the unreacted ring-opening polymerizable monomer which deteriorates moldability and thermal stability of the polymer can be simplified, or omitted.

(3) When an organic solvent is selected as a catalyst, it is not necessary to provide a step for removing the catalyst, because the core-shell type particles contain no metal catalyst.

(4) In a polymerization method using an organic solvent, it is necessary to provide a step for removing the solvent. In the method for producing core-shell type particles of the present embodiment, a drying step is simplified or omitted, because a waste liquid is not generated, and core-shell type particles in the dry form can be obtained in one-step reaction, as a compressive fluid is used.

(5) As the compressive fluid is used, a ring-opening polymerization reaction can be performed without an organic solvent.

(6) The reaction progresses uniformly because the ring-opening polymerization is carried out by adding a catalyst after the ring-opening polymerizable monomer is molten in the compressive fluid. Accordingly, the method of the present embodiment can be suitably used when copolymers with optical isomers or other monomers are produced.

Example

Examples of the present invention now will be described, but the present invention is not limited thereto in any way. In the following Examples, unless otherwise specified, "part(s)" means "part(s) by mass" and "%" means "% by mass."

<Polymerization rate of monomer>

· Polymerization rate of lactide

Nuclear magnetic resonance (NMR) spectroscopy of a polymer product constituting particles (polylactic acid) was performed in deuterated chloroform by means of a nuclear magnetic resonance apparatus (JNM-AL300, manufactured by JEOL Ltd.). In this case, a ratio of a quartet peak area derived from lactide (4.98 ppm to 5.05 ppm) to a quartet peak area derived from polylactic acid (5.10 ppm to 5.20 ppm) was calculated, followed by multiplying with 100, which was determined as an amount of an unreacted monomer (mol%). The polymerization rate is the value obtained by deducting the amount of the unreacted monomer from 100.

· Polymerization rate of $\varepsilon$-caprolactone

Nuclear magnetic resonance (NMR) spectroscopy of a polymer product constituting particles (polycaprolactone) was performed in deuterated chloroform by means of a nuclear magnetic resonance apparatus (JNM-AL300, manufactured by JEOL Ltd.). In this case, a ratio of a triplet peak area derived from caprolactone (4.22 ppm to 4.25 ppm) to a triplet peak area derived from polycaprolactone (4.04 ppm to 4.08 ppm) was calculated, followed by multiplying with 100, which was determined as an amount of an unreacted caprolactone monomer (mol%) in polycaprolactone. The polymerization rate is the value obtained by deducting the amount of the unreacted monomer from 100.

· Polymerization rate of glycolide

Nuclear magnetic resonance (NMR) spectroscopy of a polymer product constituting particles (polyglycolic acid) was performed in deuterated chloroform by means of a nuclear magnetic resonance apparatus (JNM-AL300, manufactured by JEOL Ltd.). In this case, a ratio of a triplet peak area derived from glycolide (4.25 ppm to 4.30 ppm) to a triplet peak area derived from polyglycolic acid (4.80 ppm to 4.90 ppm) was calculated, followed by multiplying with 100, which was determined as an amount of an unreacted glycolide monomer (mol%) in polyglycolic acid. The polymerization rate is the value obtained by deducting the calculated amount of the unreacted monomer from 100.

<Continuous Productivity>

After continuously operating the particle production device 1 for 8 hours or longer, the mixer 64 was disassembled, and whether or not there was any deposition of, for example, a gelation product in a one-pipe portion or a screw was visually observed. As a result, the case where there was no deposition of the gelation product was judged as "A," and the case where there were depositions of the gelation product was judged as "B."

<Particle diameter (volume average particle diameter: Dv)>

The particle size distribution was measured using MICROTRACK UPA 150 (manufactured by Nikkiso Co., Ltd.) and analyzed using an analysis software (MICROTRACK PARTICLE SIZE ANALYZER Ver. 10.1.2-016EE, manufactured by Nikkiso Co., Ltd.).

Specifically, the core-shell type particles and then water were charged in a sample glass vessel (30 mL) to thereby prepare a 10% by mass dispersion liquid. The resultant dispersion liquid was subject to dispersion treatment for 2 min by means of an ultrasonic dispersion device (W-113MK-II, manufactured by Honda Electronics Co., Ltd.). After measuring the background using water, the dispersion liquid was added dropwise and the dispersed particle diameter was measured under a condition so that the value of sample loading of a measuring device was in a range of 1 to 10. In this method, it was important that measurement was performed under the condition so that the value of sample loading of a measuring device was in a range of 1 to 10 in terms of measuring reproducibility of the dispersed particle diameter. The dropped amount of the dispersion liquid was needed to be adjusted to give the above described values of the sample loading. Measurement and analysis conditions ware set as follows.

Distribution display: volume
Selection of particle size division: standard
Number of channels: 44
Measurement time: 60 seconds
Number of measurement: 1
Transmission property of particle: transmission
Refractive index of particle: 1.5
Particle shape: non-spherical
Density: 1 g/cm$^3$

<BET Specific surface area>

The BET specific surface area of the core-shell type particles was measured with an automatic specific surface area/pore distribution measuring device TRISTAR 3000 (manufactured by SHIMADZU CORPORATION). One gram of the core-shell type particles was placed in a dedicated cell, and the inside of the dedicated cell was degassed using a degassing dedicated unit for TRISTAR, VACUPREP 061 (manufactured by SHIMADZU CORPORATION). The degassing treatment was carried out at room temperature at least for 20 hours under reduced pressure of 100 mtorr or less. The dedicated cell which had been subjected to the degassing treatment could be automatically subjected to the BET specific surface area measurement with TRISTAR 3000. Note that, nitrogen gas was used as adsorption gas.

<Average pore diameter>

The particles (porous particles or core-shell type particles) were observed by means of a field emission scanning electron microscope (FE-SEM). The pore diameters of pores of the particles were measured based on the resultant images. The pore diameters were measured for 100 pores and averaged, which was determined as an average pore diameter.

<Molecular weight>

The molecular weight was measured through gel permeation chromatography (GPC) under the following conditions.

Apparatus: GPC-8020 (manufactured by TOSOH CORPORATION)
Column: TSK G2000HXL and G4000HXL (manufactured by TOSOH CORPORATION)
Temperature: 40°C
Solvent: Tetrahydrofurane (THF)
Flow rate: 1.0 mL/min

First, a calibration curve of molecular weight was obtained using monodispersed polystyrene serving as a standard sample. A polymer sample (1 mL) having a concentration of 0.5% by mass was loaded and measured under the above conditions, to thereby obtain the molecular weight distribution of the polymer. Based on the molecular weight distribution, the number average molecular weight (Mn) and the weight average molecular weight (Mw) of the polymer were calculated from the calibration curve. The molecular weight distribution is a value calculated by dividing Mw with Mn.

<Average thickness of core-shell type particles and shell layer>

It was confirmed that the resultant particles were core-shell type particles by observing cross-sections of the particles by means of a scanning electron microscope.
The thickness of the shell layer of each of the core-shell type particle was measured with a ruler based on images obtained by observing cross-sections of the core-shell type particles by means of the scanning electron microscope. The thicknesses of the shell layers were measured at 100 points and averaged, which was determined as the average thickness of the shell layer.

(Example 1)

Particles each in which a surface of porous apatite was coated with a polymer obtained through ring-opening polymerization of L-lactide (hereinafter may be referred to as "lactide") were produced using the particle production device 1 of FIG. 3. A $CO_2$ (carbon dioxide) bomb was used as the bomb 21. A nitrogen bomb was used as the bomb 51. Note that, in Example 1, the additive tank 41, the pump 42, and the mixer 67 were not used.
The lactide serving as the ring-opening polymerizable monomer and porous apatite (particle diameter: 6.0 $\mu$m, hydroxyapatite, mSHAp, manufactured by SofSera Corporation) were charged into the tank 11 of the particle production device 1 shown in FIG. 3, followed by heat-melting. Lauryl alcohol serving as the initiator was charged into the tank 11 so that a molar ratio of the lauryl alcohol to the lactide is 1:99. The pump 22 was activated to open the valve 23, to thereby introduce carbon dioxide serving as the first compressive fluid at 40°C and 50 MPa. The pump 12 was activated to open the valve 13, to thereby continuously bring the first compressive fluid into contact with raw material which is a mixture of the lactide, the porous apatite, and the lauryl alcohol in the tank 11. The resultant was mixed by the mixer 64 (static mixer) to thereby obtain Mixture A. Here, the raw material was supplied to the mixer 64 at 190 g/min, and the first compressive fluid was supplied to the mixer 64 at 10 g/min.
Next, the pump 32 was activated to open the valve 33, to thereby supply the catalyst 1,8-diazabicyclo[5.4.0]undec-7-ene (DBU) charged into the catalyst tank 31 to the mixer 65 (static mixer) so that a molar ratio of the DBU to the lactide is 0.1:99.9. The resultant was mixed with Mixture A to thereby obtain Mixture B. The resultant Mixture B was introduced into the reaction container 66 (tubular reactor), where the lactide was allowed to ring-opening polymerize to thereby obtain Mixture C containing a polymer product. An average retention time of Mixtures B and C in the reaction container 66 was set to about 20 min.
Next, using the pump 52 and the heater 61, the resultant Mixture C was continuously injected from the nozzle 69 having a nozzle diameter of 100 $\mu$m while supercritical nitrogen serving as the second compressive fluid was supplied to Mixture C so that the temperature and pressure were kept at 40°C and 50 MPa, respectively. The injected Mixture C was formed into particles, followed by solidifying to thereby obtain [Core-shell type particles 1].

The polymerization rate and continuous productivity in Example 1 are shown in Tables 1-1-1 and 1-1-2.

The volume average particle diameter Dv, average pore diameter, BET specific surface area, and average thickness of shell layers of [Core-shell type particles 1] are shown in Table 1-3.

The weight average molecular weight Mw and molecular weight distribution (Mw/Mn) of the polymer constituting [Core-shell type particles 1] are shown in Table 1-1-2.

<Fine coatability>

The resultant core-shell type particles was observed by means of SEM to determine whether each of the porous particles serving as a core was uniformly coated with the polymer serving as a shell layer so as to conform to fine structures on the surface of each of the porous particles, and evaluated according to the following criteria. Results are shown in Table 1-3.

[Evaluation]

A: Inside of pores of porous particles was coated.
B: Pores of porous particles were clogged with a coating film.

(Examples 2 to 19)

Core-shell type particles were obtained in the same manner as in Example 1, except that the types, physical properties, and injection conditions (nozzle diameter) of monomers and porous particles were changed to those described in Tables 1-1-1 and 1-1-2. The resultant core-shell type particles were evaluated in the same manner as in Example 1. Results are shown in Tables 1-1-2, and 1-3.

(Comparative Example 1)

To a 300 mL four-necked separable flask, were added 200 g of L-lactide and 10 g of porous apatite (particle diameter: 6.0 $\mu$m, hydroxyapatite, mSHAp, manufactured by SofSera Corporation), followed by gradually heating until the internal temperature thereof is 150°C, and dehydrating at 10 mmHg for 30 min. Then, the resultant was heated to 170°C while purging with $N_2$. Uniformity of the system was visually confirmed. Thereafter, 50 mg of tin 2-ethylhexanoate was charged into the system to thereby allow them to polymerize, during which the internal temperature of the system was controlled to 190°C or lower. After 2 hours of a reaction time passed, the system was re-switched to an efflux line, and the lactide was removed under conditions at 190°C and 10 mmHg. Thus, the polymerization reaction was completed and an organic-inorganic complex was obtained. Then, the organic-inorganic complex was left to stand under an environment of 40°C and 50 MPa for 2 hours in the same manner as in Example 1, and the mixture was continuously injected from the nozzle 69 having a nozzle diameter of 400 $\mu$m. The injected mixture was formed into particles, followed by solidifying to thereby obtain [Core-shell type particles]. However, in [Core-shell type particles], the porous apatite was not uniformly coated with the polymer so as to conform to fine structures on the surface of the porous apatite.

(Comparative Example 2)

The organic-inorganic complex synthesized in Comparative Example 1 was dissolved into ethyl acetate, which was used as an oil phase. An aqueous phase was separately prepared. Emulsification was performed using the oil phase and the aqueous phase. Note that, an ionic surfactant, an emulsification stabilizer, and a thickener were added into the aqueous phase in a predetermined amount. As a result, the surface of the 6.0 $\mu$m porous apatite was ununiformly coated with polylactic acid.

Table 1-1-1

| | | Porous particles | | | Polymer (coating agent) | |
|---|---|---|---|---|---|---|
| | Type | Volume average particle diameter Dv ($\mu$m) | Average pore diameter (nm) | BET specific surface area ($m^2$/g) | Type of monomer | Polymerization rate (% by mol) |
| Example 1 | Hydroxyapatite | 6.0 | 100 | 13.2 | L-Lactide | 99.3 |
| Example 2 | Hydroxyapatite | 6.0 | 100 | 13.2 | L-Lactide | 98.7 |
| Example 3 | Hydroxyapatite | 6.0 | 100 | 13.2 | L-Lactide | 99.4 |
| Example 4 | Hydroxyapatite | 6.0 | 100 | 13.2 | L-Lactide | 99.3 |
| Example 5 | Hydroxyapatite | 6.0 | 100 | 13.2 | L-Lactide | 99.3 |
| Example 6 | Hydroxyapatite | 3.0 | 100 | 16.4 | L-Lactide | 99.3 |
| Example 7 | Hydroxyapatite | 10.0 | 100 | 10.7 | L-Lactide | 99.3 |
| Example 8 | Hydroxyapatite | 6.0 | 150 | 11.6 | L-Lactide | 99.3 |
| Example 9 | Hydroxyapatite | 6.0 | 70 | 15.8 | L-Lactide | 99.3 |
| Example 10 | $\beta$-Tricalcium phosphate | 4.7 | 120 | 12.1 | L-Lactide | 99.3 |
| Example 11 | $\alpha$-Tricalcium phosphate | 4.5 | 120 | 12.5 | L-Lactide | 99.3 |
| Example 12 | Octacalcium phosphate | 5.2 | 140 | 12.0 | L-Lactide | 99.3 |
| Example 13 | BMP Protein | 0.6 | 60 | 24.3 | L-Lactide | 99.3 |
| Example 14 | Silica | 1.8 | 125 | 17.2 | L-Lactide | 99.3 |
| Example 15 | Zirconia | 1.3 | 130 | 19.7 | L-Lactide | 99.3 |
| Example 16 | Alumina | 2.2 | 125 | 16.5 | L-Lactide | 99.3 |
| Example 17 | Zincite | 1.5 | 120 | 19.1 | L-Lactide | 99.3 |
| Example 18 | Hydroxyapatite | 6.0 | 100 | 13.2 | $\varepsilon$-caprolactone | 99.7 |
| Example 19 | Hydroxyapatite | 6.0 | 100 | 13.2 | Glycolide | 98.8 |
| Comparative Example 1 | Hydroxyapatite | 6.0 | 100 | 13.2 | L-Lactide | 99.3 |
| Comparative Example 2 | Hydroxyapatite | 6.0 | 100 | 13.2 | L-Lactide | 99.3 |

Table 1-1-2

| | Polymer (Coating Agent) | | Step | | |
|---|---|---|---|---|---|
| | Mw | Mw/Mn | Nozzle diameter ($\mu$m) | Continuously with polymerization step | Continuous productivity |
| Example 1 | 50,000 | 1.82 | 100 | Continuous | A |
| Example 2 | 5,000 | 1.68 | 100 | Continuous | A |
| Example 3 | 200,000 | 1.88 | 100 | Continuous | A |

(continued)

| | Polymer (Coating Agent) | | Step | | |
|---|---|---|---|---|---|
| | Mw | Mw/Mn | Nozzle diameter (μm) | Continuously with polymerization step | Continuous productivity |
| Example 4 | 50,000 | 1.82 | 150 | Continuous | A |
| Example 5 | 50,000 | 1.82 | 50 | Continuous | A |
| Example 6 | 50,000 | 1.82 | 100 | Continuous | A |
| Example 7 | 50,000 | 1.82 | 100 | Continuous | A |
| Example 8 | 50,000 | 1.82 | 100 | Continuous | A |
| Example 9 | 50,000 | 1.82 | 100 | Continuous | A |
| Example 10 | 50,000 | 1.82 | 100 | Continuous | A |
| Example 11 | 50,000 | 1.82 | 100 | Continuous | A |
| Example 12 | 50,000 | 1.82 | 100 | Continuous | A |
| Example 13 | 50,000 | 1.82 | 100 | Continuous | A |
| Example 14 | 50,000 | 1.82 | 100 | Continuous | A |
| Example 15 | 50,000 | 1.82 | 100 | Continuous | A |
| Example 16 | 50,000 | 1.82 | 100 | Continuous | A |
| Example 17 | 50,000 | 1.82 | 100 | Continuous | A |
| Example 18 | 32,000 | 1.57 | 100 | Continuous | A |
| Example 19 | 43,000 | 1.62 | 100 | Continuous | A |
| Comparative Example 1 | 50,000 | 1.82 | 100 | Non-Continuous | A |
| Comparative Example 2 | 50,000 | 1.82 | 100 | Non-Continuous | A |

In Table 1-1-1, the types of porous particles are as follows.

Table 1-2

| Porous particles | | | | | |
|---|---|---|---|---|---|
| Type | Volume average particle diameter Dv (μm) | Average pore diameter (nm) | BET specific surface area (m$^2$/g) | Trade name | Supplier name |
| Hydroxyapatite | 6.0 | 100 | 13.2 | mSHAp | SofSera Corporation |
| Hydroxyapatite | 3.0 | 100 | 16.4 | mSHAp | SofSera Corporation |
| Hydroxyapatite | 10.0 | 100 | 10.7 | mSHAp | SofSera Corporation |
| Hydroxyapatite | 6.0 | 150 | 11.6 | mSHAp | SofSera Corporation |
| Hydroxyapatite | 6.0 | 70 | 15.8 | mSHAp | SofSera Corporation |
| β-Tricalcium phosphate | 4.7 | 120 | 12.1 | β-TCP-100 | TAIHEI CHEMICAL INDUSTRIAL CO., LTD. |
| α-Tricalcium phosphate | 4.5 | 120 | 12.5 | α-TCP | TAIHEI CHEMICAL INDUSTRIAL CO., LTD. |

(continued)

| Porous particles | | | | | |
|---|---|---|---|---|---|
| Type | Volume average particle diameter Dv ($\mu$m) | Average pore diameter (nm) | BET specific surface area ($m^2$/g) | Trade name | Supplier name |
| Octacalcium phosphate | 5.2 | 140 | 12.0 | OCP | TAIHEI CHEMICAL INDUSTRIAL CO., LTD. |
| BMP Protein | 0.6 | 60 | 24.3 | - | - |
| Silica | 1.8 | 125 | 17.2 | - | - |
| Zirconia | 1.3 | 130 | 19.7 | - | - |
| Alumina | 2.2 | 125 | 16.5 | - | - |
| Zincite | 1.5 | 120 | 19.1 | - | - |

Table 1-3

| | Core-shell type particles | | | | |
|---|---|---|---|---|---|
| | Volume average particle diameter Dv ($\mu$m) | Average pore diameter (nm) | BET specific surface area ($m^2$/g) | Average thickness of shell layer (nm) | Fine coatability |
| Example 1 | 6.0 | 62 | 12.8 | 18 | A |
| Example 2 | 6.0 | 80 | 13.1 | 10 | A |
| Example 3 | 6.0 | 18 | 12.5 | 42 | A |
| Example 4 | 6.0 | 47 | 12.6 | 29 | A |
| Example 5 | 6.0 | 85 | 13.0 | 8 | A |
| Example 6 | 3.0 | 64 | 16.0 | 17 | A |
| Example 7 | 10.0 | 63 | 10.3 | 18 | A |
| Example 8 | 6.0 | 111 | 11.1 | 18 | A |
| Example 9 | 6.0 | 40 | 14.9 | 17 | A |
| Example 10 | 4.7 | 86 | 11.5 | 21 | A |
| Example 11 | 4.5 | 78 | 11.9 | 23 | A |
| Example 12 | 5.2 | 99 | 11.5 | 20 | A |
| Example 13 | 0.6 | 21 | 21.6 | 19 | A |
| Example 14 | 1.8 | 86 | 16.8 | 17 | A |
| Example 15 | 1.3 | 89 | 19.2 | 18 | A |
| Example 16 | 2.2 | 82 | 15.9 | 17 | A |
| Example 17 | 1.5 | 81 | 18.7 | 19 | A |
| Example 18 | 6.0 | 66 | 13.0 | 15 | A |
| Example 19 | 6.0 | 60 | 13.0 | 18 | A |
| Comparative Example 1 | 6.0 | 0 | 1.6 | - | B |
| Comparative Example 2 | 6.0 | 0 | 1.4 | - | B |

Embodiments of the present invention are as follows.

<1> A method for producing core-shell type particles, including:

ring-opening polymerizing a ring-opening polymerizable monomer in a first mixture containing the ring-opening polymerizable monomer, porous particles, and a compressive fluid; and
injecting a second mixture containing a polymer obtained in the ring-opening polymerizing, the porous particles, and the compressive fluid to thereby granulate into the core-shell type particles.

<2> The method for producing core-shell type particles according to <1>, wherein the first mixture contains a catalyst.
<3> The method for producing core-shell type particles according to <1> or <2>, wherein the compressive fluid contains carbon dioxide.
<4> The method for producing core-shell type particles according to any one of <1> to <3>, wherein the ring-opening polymerizable monomer is a monomer having a ring structure containing a carbonyl group.
<5> The method for producing core-shell type particles according to any one of <1> to <4>, wherein the porous particles are protein, calcium phosphate, metal oxide, or any combination thereof.
<6> The method for producing core-shell type particles according to <5>, wherein the protein is BMP, EP4A, FGF-18, VEGF, bFGF, or any combination thereof.
<7> The method for producing core-shell type particles according to <5>, wherein the calcium phosphate is hydroxyapatite, α-tricalcium phosphate, β-tricalcium phosphate, tetracalcium phosphate, octacalcium phosphate, or any combination thereof.
<8> The method for producing core-shell type particles according to <5>, wherein the metal oxide is silica, titania, zirconia, zincite, or any combination thereof.
<9> Core-shell type particles produced by the method for producing core-shell type particles according to any one of <1> to <8>.
<10> Core-shell type particles, each including:

a core particle which is a porous particle; and
a shell layer which coats the core particle,
wherein the core-shell type particles have a volume average particle diameter (Dv) of 0.1 μm to 20.0 μm,
wherein the core-shell type particles have an average pore diameter of 10 nm to 200 nm,
wherein the core-shell type particles have a BET specific surface area of 1 $m^2$/g to 100 $m^2$/g, and
wherein the shell layers of the core-shell type particles have an average thickness of 10 nm to 3 μm.

<11> The core-shell type particles according to <10>, wherein a difference in the BET specific surface area between the core-shell type particles and the core particles is 1.5 $m^2$/g or less in terms of an absolute value.
<12> The core-shell type particles according to <10> or <11>, wherein a difference in the average pore diameter between the core-shell type particles and the core particles is 100 nm or less in terms of an absolute value.

**Claims**

1. A method for producing core-shell type particles, comprising:

ring-opening polymerizing a ring-opening polymerizable monomer in a first mixture containing the ring-opening polymerizable monomer, porous particles, and a compressive fluid; and
injecting a second mixture containing a polymer obtained in the ring-opening polymerizing, the porous particles, and the compressive fluid to thereby granulate into the core-shell type particles.

2. The method for producing core-shell type particles according to claim 1, wherein the first mixture contains a catalyst.

3. The method for producing core-shell type particles according to claim 1 or 2, wherein the compressive fluid contains carbon dioxide.

4. The method for producing core-shell type particles according to any one of claims 1 to 3, wherein the ring-opening polymerizable monomer is a monomer having a ring structure containing a carbonyl group.

5. The method for producing core-shell type particles according to any one of claims 1 to 4, wherein the porous particles are protein, calcium phosphate, metal oxide, or any combination thereof.

6. The method for producing core-shell type particles according to claim 5, wherein the protein is BMP, EP4A, FGF-18, VEGF, bFGF, or any combination thereof.

7. The method for producing core-shell type particles according to claim 5, wherein the calcium phosphate is hydroxyapatite, $\alpha$-tricalcium phosphate, $\beta$-tricalcium phosphate, tetracalcium phosphate, octacalcium phosphate, or any combination thereof.

8. The method for producing core-shell type particles according to claim 5, wherein the metal oxide is silica, titania, zirconia, zincite, or any combination thereof.

9. Core-shell type particles produced by the method for producing core-shell type particles according to any one of claims claims 1 to 8.

10. Core-shell type particles, each comprising:

a core particle which is a porous particle; and
a shell layer which coats the core particle,
wherein the core-shell type particles have a volume average particle diameter (Dv) of 0.1 $\mu$m to 20.0 $\mu$m,
wherein the core-shell type particles have an average pore diameter of 10 nm to 200 nm,
wherein the core-shell type particles have a BET specific surface area of 1 m$^2$/g to 100 m$^2$/g, and
wherein the shell layers of the core-shell type particles have an average thickness of 10 nm to 3 $\mu$m.

11. The core-shell type particles according to claim 10, wherein a difference in the BET specific surface area between the core-shell type particles and the core particles is 1.5 m$^2$/g or less in terms of an absolute value.

12. The core-shell type particles according to claim 10 or 11, wherein a difference in the average pore diameter between the core-shell type particles and the core particles is 100 nm or less in terms of an absolute value.

# FIG. 1

# FIG. 2

# FIG. 3

1

A          B

31          41          52   53

51

He

21          33          42
22  23      32          43          68          69
Hb          Hc          Hd
64b
64
64a                                              61
11          65   Hf   66   67
12  13
Ha

P

# FIG. 4

| System 1 | System 2 |
| --- | --- |

2

PP

# FIG. 5

# FIG. 6

Europäisches
Patentamt

European
Patent Office

Office européen
des brevets

## EUROPEAN SEARCH REPORT

Application Number

EP 14 17 0164

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KAVITHA KOUSHIK ET AL: "Preparation of Large Porous Deslorelin PLGA Microparticles with Reduced Residual Solvent and Cellular Uptake Using a Supercritical Carbon Dioxide Process", PHARMACEUTICAL RESEARCH, SPRINGER NEW YORK LLC, US, vol. 21, no. 3, 1 March 2004 (2004-03-01), pages 524-535, XP007905372, ISSN: 0724-8741, DOI: 10.1023/B:PHAM.0000019308.25479.A4 * page 524, right-hand column - page 534, left-hand column, line 16 * | 1-12 | INV. C08J3/12 |
| A | MISHIMA ET AL: "Biodegradable particle formation for drug and gene delivery using supercritical fluid and dense gas", ADVANCED DRUG DELIVERY REVIEWS, ELSEVIER, AMSTERDAM, NL, vol. 60, no. 3, 11 October 2007 (2007-10-11), pages 411-432, XP022476833, ISSN: 0169-409X, DOI: 10.1016/J.ADDR.2007.02.003 * the whole document * | 1-12 | |
| A | EP 2 497 793 A2 (TYCO HEALTHCARE [US] COVIDIEN LP [US]) 12 September 2012 (2012-09-12) * paragraphs [0007] - [0045]; examples * * paragraphs [0009] - [0011] * | 1-12 | TECHNICAL FIELDS SEARCHED (IPC)<br><br>C08J |

-/--

The present search report has been drawn up for all claims

| Place of search | Date of completion of the search | Examiner |
|---|---|---|
| Munich | 25 September 2014 | Hutton, David |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 14 17 0164

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KIM J-H ET AL: "Microencapsulation of Naproxen using rapid expansion of supercritical solutions", BIOTECHNOLOGY PROGRESS, AMERICAN INSTITUTE OF CHEMICAL ENGINEERS, US, vol. 12, no. 5, 1 January 1996 (1996-01-01), pages 650-661, XP002342389, ISSN: 8756-7938, DOI: 10.1021/BP9600492 * the whole document * | 1-12 | |
| A | ANDREW I. COOPER: "Polymer synthesis and processing using supercritical carbon dioxide", JOURNAL OF MATERIALS CHEMISTRY, vol. 10, no. 2, 1 January 2000 (2000-01-01), pages 207-234, XP055112373, ISSN: 0959-9428, DOI: 10.1039/a906486i * the whole document * | 1-12 | |
| A | WO 2013/018873 A1 (RICOH CO LTD [JP]; NEMOTO TAICHI [JP]; TANAKA CHIAKI [JP]; YAMAUCHI YO) 7 February 2013 (2013-02-07) * the whole document * * page 14, lines 7-21; examples * | 1-12 | TECHNICAL FIELDS SEARCHED (IPC) |
| A | WO 2013/024834 A1 (RICOH CO LTD [JP]; YAMAUCHI YOSHITAKA [JP]; TANAKA CHIAKI [JP]; NEMOTO) 21 February 2013 (2013-02-21) * claims; examples 5-1,5-11-5-13 * | 1-12 | |
| A | WO 2013/018874 A1 (RICOH CO LTD [JP]; NEMOTO TAICHI [JP]; TANAKA CHIAKI [JP]; YAMAUCHI YO) 7 February 2013 (2013-02-07) * the whole document * | 1-12 | |

-/--

The present search report has been drawn up for all claims

| Place of search | Date of completion of the search | Examiner |
|---|---|---|
| Munich | 25 September 2014 | Hutton, David |

EPO FORM 1503 03.82 (P04C01)

Europäisches
Patentamt

European
Patent Office

Office européen
des brevets

**EUROPEAN SEARCH REPORT**

Application Number

EP 14 17 0164

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | VERÓNICA LASSALLE ET AL: "PLA Nano- and Microparticles for Drug Delivery: An Overview of the Methods of Preparation", MACROMOLECULAR BIOSCIENCE, vol. 7, no. 6, 7 June 2007 (2007-06-07), pages 767-783, XP055116551, ISSN: 1616-5187, DOI: 10.1002/mabi.200700022 * the whole document * * page 774, right-hand column, paragraph 2 * | 1-12 | |

TECHNICAL FIELDS
SEARCHED (IPC)

The present search report has been drawn up for all claims

| Place of search | Date of completion of the search | Examiner |
|---|---|---|
| Munich | 25 September 2014 | Hutton, David |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 14 17 0164

This annex lists the patent family members relating to the patent documents cited in the above-mentioned European search report.
The members are as contained in the European Patent Office EDP file on
The European Patent Office is in no way liable for these particulars which are merely given for the purpose of information.

25-09-2014

| Patent document cited in search report | | Publication date | Patent family member(s) | | Publication date |
|---|---|---|---|---|---|
| EP 2497793 | A2 | 12-09-2012 | AU | 2012201389 A1 | 20-09-2012 |
| | | | CA | 2770169 A1 | 11-09-2012 |
| | | | EP | 2497793 A2 | 12-09-2012 |
| | | | JP | 2012188664 A | 04-10-2012 |
| | | | US | 2012231165 A1 | 13-09-2012 |
| WO 2013018873 | A1 | 07-02-2013 | CA | 2843239 A1 | 07-02-2013 |
| | | | CN | 103857726 A | 11-06-2014 |
| | | | EP | 2736943 A1 | 04-06-2014 |
| | | | JP | 2013216851 A | 24-10-2013 |
| | | | KR | 20140043458 A | 09-04-2014 |
| | | | WO | 2013018873 A1 | 07-02-2013 |
| WO 2013024834 | A1 | 21-02-2013 | AU | 2012295894 A1 | 06-03-2014 |
| | | | CA | 2844896 A1 | 21-02-2013 |
| | | | CN | 103732649 A | 16-04-2014 |
| | | | EP | 2742081 A1 | 18-06-2014 |
| | | | JP | 2013224398 A | 31-10-2013 |
| | | | KR | 20140058620 A | 14-05-2014 |
| | | | US | 2014200326 A1 | 17-07-2014 |
| | | | WO | 2013024834 A1 | 21-02-2013 |
| WO 2013018874 | A1 | 07-02-2013 | CA | 2843391 A1 | 07-02-2013 |
| | | | CN | 103827168 A | 28-05-2014 |
| | | | EP | 2736944 A1 | 04-06-2014 |
| | | | JP | 2013189613 A | 26-09-2013 |
| | | | KR | 20140048285 A | 23-04-2014 |
| | | | US | 2014163194 A1 | 12-06-2014 |
| | | | WO | 2013018874 A1 | 07-02-2013 |

EPO FORM P0459

For more details about this annex : see Official Journal of the European Patent Office, No. 12/82

**REFERENCES CITED IN THE DESCRIPTION**

**Patent documents cited in the description**

- JP 2006104342 A
- JP 3270198 A
- JP 11506744 A
- JP 47015526 A
- JP 47015527 A
- JP 47015528 A
- JP 47015533 A
- JP 47033166 A